# EUROPÄISCHE PATENTSCHRIFT

(11) **EP 1 957 656 B1**
(45) Veröffentlichungstag und Bekanntmachung des Hinweises auf die Patenterteilung: **30.04.2014**
(21) Anmeldenummer: 06830135.7
(22) Anmeldetag: 27.11.2006
(51) Int. Cl.: C12P 13/08, C12N 1/20

(54) **Fermentative Herstellung von Lysin**
Fermentative production of lysine
Production de lysine par fermentation

(30) Priorität: 28.11.2005 DE 102005056668
(43) Veröffentlichungstag der Anmeldung: 20.08.2008
(73) Patentinhaber: BASF SE, 67056 Ludwigshafen (DE)
(72) Erfinder: POMPEJUS, Markus, Seoul 140-210 (KR); FREYER, Stephan, 67434 Neustadt (DE); LOHSCHEIDT, Markus, 69121 Heidelberg (DE); ZELDER, Oskar, 67346 Speyer (DE); BOY, Matthias, 63225 Langen (DE)
(86) Internationale Anmeldenummer: PCT/EP2006/068926
(87) Internationale Veröffentlichungsnummer: WO 2007/060233

(56) Entgegenhaltungen:
- WO-A-98/55599
- WO-A-02/066663
- WO-A-2004/113551
- WO-A-2005/059144
- WO-A2-2005/021771
- WO-A2-2005/116228
- DE-A1- 3 146 558
- DE-A1- 3 731 293
- DE-A1- 19 519 270
- US-A- 3 787 587
- US-A- 4 306 023
- US-A- 5 503 750
- DATABASE WPI Week 200216 Derwent Publications Ltd., London, GB; AN 2002-117450 XP002426324 & JP 2001 275693 A (AJINOMOTO CO INC) 9. Oktober 2001 (2001-10-09) in der Anmeldung erwähnt
- PFEFFERLE W. ET AL.: "Biotechnological manufacture of Lysine", ADVANCES IN BIOCHEMICAL ENGINEERING/BIOTECHNOLOGY, vol. 79, 1 January 2003 (2003-01-01), pages 59-112, BERLIN
- Voet D und Voet JG: "Biochemie", 1990, VCH Weinheim ISBN: 3-527-29249-7 page 850,
- CHRISTIAN JUNG, STEFAN GRÄBER: 'Getreide' RÖMPP ONLINE, VERSION 3.9, [Online] Seiten 1 - 1 Gefunden im Internet: <URL:http://www.roempp.com/prod/roempp.php> [gefunden am 2010-11-24]
- ANONYMOUS: 'Begleitalkohol' WIKIPEDIA, [Online] Seiten 1 - 3 Gefunden im Internet: <URL:http://de.wikipedia.org/wiki/Begleital kohol> [gefunden am 2010-12-13]
- Anonymous: "Bioethanol - Produktionsverfahren", Crop Energies AG, 6 August 2009 (2009-08-06), pages 1-4, Retrieved from the Internet: URL:http://www.cropenergies.com/de/Bioetha nol/Produktionsverfahren/

## Beschreibung

Die vorliegende Erfindung betrifft die fermentative Herstellung von Lysin unter Einsatz eines zuckerhaltigen Mediums, das zumindest einen Teil der nicht-stärkehaltigen festen Bestandteile der Stärkequelle umfasst, zur Kultivierung der Mikroorganismen.

Zuckerhaltige Flüssigmedien sind eine grundlegende Nährstoffquelle für viele Fermentationsverfahren; die in den Medien enthaltenen Zuckeranteile werden von den eingesetzten Mikroorganismen verstoffwechselt, wobei man organische Wertprodukte erhält. Die Palette derart hergestellter mikrobieller Stoffwechselprodukte, d. h. organischer Verbindungen, umfasst hierbei z. B. niedermolekulare flüchtige Verbindungen wie Ethanol, nichtflüchtige Stoffwechselprodukte wie Aminosäuren, Vitamine und Carotenoide sowie eine Vielzahl weiterer Stoffe.

Für solche allgemein bekannten mikrobiellen Fermentationsverfahren werden in Abhängigkeit von den verschiedenen Verfahrensbedingungen unterschiedliche Kohlenstoffquellen genutzt. Diese reichen von reiner Saccharose über Rüben- und Zuckerrohrmelasse, sogenannten "high test molasses" (invertierte Zuckerrohrmelasse) bis hin zu Glucose aus Stärkehydrolysaten. Für die biotechnologische Herstellung von L-Lysin werden darüber hinaus Essigsäure und Ethanol als großtechnisch einsetzbare Co-Substrate genannt (Pfefferle et al., Biotechnogical Manufacture of Lysine, Advances in Biochemical Engineering/Biotechnology, Vol. 79 (2003), 59-112).

Auf Basis der genannten Kohlenstoffquellen sind verschiedene Methoden und Vorgehensweisen zur zuckerbasierten, fermentativen Herstellung mikrobieller Stoffwechselprodukte etabliert. Am Beispiel des L-Lysins werden diese beispielsweise von Pfefferle et al. (a.a.O.) im Hinblick auf Stammentwicklung, Prozessentwicklung und großtechnische Produktion beschrieben.

Eine wichtige Kohlenstoffquelle für die durch Mikroorganismen vermittelte fermentative Herstellung mikrobieller Stoffwechselprodukte ist Stärke. Diese muss in vorgelagerten Reaktionsschritten zunächst verflüssigt und verzuckert werden, bevor sie als Kohlenstoffquelle in einer Fermentation genutzt werden kann. Hierzu wird die Stärke aus einer natürlichen Stärkequelle wie Kartoffeln, Cassava, Getreide, z. B. Weizen, Mais, Gerste, Roggen, Triticale oder Reis üblicherweise in vorgereinigter Form gewonnen und anschließend enzymatisch verflüssigt und verzuckert, um dann in der eigentlichen Fermentation zur Produktion der gewünschten Stoffwechselprodukte eingesetzt zu werden.

Neben dem Einsatz derartig vorgereinigter Stärkequellen ist auch die Verwendung nicht aufgereinigter Stärkequellen zur Herstellung von Kohlenstoffquellen für die fermentative Produktion mikrobieller Stoffwechselprodukte beschrieben worden. Typischerweise werden die Stärkequellen dabei zunächst durch Mahlen zerkleinert. Das Mahlgut wird dann einer Verflüssigung und Verzuckerung unterworfen. Da dieses Mahlgut naturgemäß neben Stärke noch eine Reihe von nicht-stärkehaltigen Bestandteilen enthält, welche die Fermentation nachteilig beeinflussen können, werden diese Bestandteile üblicherweise vor der Fermentation abgetrennt. Die Entfernung kann entweder direkt nach dem Mahlen (WO 02/077252; JP 2001-072701; JP 56-169594;

CN 1218111), nach der Verflüssigung (WO 02/077252; CN 1173541) oder im Anschluss an die Verzuckerung (CN 1266102; Beukema et al.: Production of fermentation syrups by enzymatic hydrolysis of potatoes; potatoe saccharification to give culture medium (Conference Abstract), Symp. Biotechnol. Res. Neth. (1983), 6; NL8302229) erfolgen. In allen Varianten wird jedoch ein weitgehend reines Stärkehydrolysat in der Fermentation eingesetzt.

Demgegenüber wird in US 5,503,750 ein speziell für die Herstellung von Milchsäure entwickeltes Fermentationsverfahren unter Verwendung von verzuckerter Getreidemaische beschrieben, bei dem die Michsäure durch Ultrafiltration und nachfolgende Nanofiltration kontinuierlich abgetrennt und so aus dem Fermenter entfernt wird. Die Retentate der Ultra- und Nanofiltration, welche nicht-fermentierten Restzucker enthalten, werden in den Fermenter zurückgeführt.

Neuere Verfahren zur fermentativen Herstellung von organischen Verbindungen umfassen insbesondere eine Aufreinigung der Stärkequellen vor der Fermentation z. B. die Aufreinigung von verflüssigten und verzuckerten Stärkelösungen (JP 57159500), oder stellen Methoden bereit, welche die Herstellung von Fermentationsmedien aus erneuerbaren Ressourcen (EP 1205557) ermöglichen sollen.

Nicht aufgereinigte Stärkequellen hingegen werden in großem Umfang bei der fermentativen Herstellung von Bioethanol eingesetzt. Hierbei werden die Stärkequellen, üblicherweise ganze Getreidekörner, zunächst trocken vermahlen und anschließend der Stärkebestandteil der Stärkequelle unter Einwirkungen von Enzymen hydrolysiert. Dabei kann die Hydrolyse sowohl diskontinuierlich, z. B. in Rührkesseln, als auch kontinuierlich, z. B. In Jet-Kochern durchgeführt werden. Entsprechende Prozessbeschreibungen finden sich z. B. in "The Alcohol Textbook - A reference for the beverage, fuel and industrial alcohol industries", Jaques et al. (Hg.), Nottingham Univ. Press 1995, ISBN 1-8977676-735, Kapitel 2, S. 7 bis 23, und in McAloon et al., "Determining the cost of producing ethanol from corn starch and lignocellulosic feedstocks", NREL/TP-580-28893, National Renewable Energy Laboratory, October 2000.

Da bei der fermentativen Herstellung von Bioethanol das Wertprodukt durch Destillation gewonnen wird, stellt der Einsatz von Stärkequellen aus dem Dry-Milling-Prozess in nicht vorgereinigter Form kein gravierendes Problem dar. Bei der Anwendung eines Dry-Milling-Verfahrens zur Herstellung anderer mikrobieller Stoffwechselprodukte ist jedoch der über die Zuckerlösung in die Fermentation eingetragene Feststoffstrom problematisch, da dieser sich sowohl negativ auf die Fermentation auswirken kann, z. B. im Hinblick auf die Sauerstofftransferrate bzw. den Sauerstoffbedarf der eingesetzten Mikroorganismen (vgl. hierzu Mersmann, A. et al.: Selection and Design of Aerobic Bioreactors, Chem. Eng. Technol. 13 (1990), 357-370) als auch die anschließende Aufarbeitung nicht unerheblich erschweren kann.

Zudem kann durch den Feststoffeintrag bereits bei der Herstellung der stärkehaltigen Suspension die Viskosität der Suspension einen kritischen Wert erreichen, wodurch z. B. eine Suspension mit mehr als 30 Gew.-% Maismehl in Wasser nicht mehr homogen mischbar ist (Industrial Enzymology, 2. Aufl., T. Godfrey, S. West, 1996). Dadurch ist bei herkömmlichem Vorgehen die Glucosekonzentration begrenzt. Dies ist im Hinblick auf die fermentative Bioethanol-Herstellung insofern nicht weiter relevant, da höhere Konzentrationen aufgrund der Toxizität des Produkts für die zur Fermentation eingesetzten Hefen ohnehin nicht sinnvoll umgesetzt werden könnten.

Bei der fermentativen Herstellung anderer organischer Stoffwechselprodukte als Ethanol ist es prinzipiell von Nachteil, der Fermentation zuckerhaltige Medien mit geringer Zuckerkonzentrationen zuzuführen, weil dies zu einer überproportionalen Verdünnung der Fermentationsbrühe führt und sich folglich die erreichbare Endkonzentration der Wertprodukte verringert, was einerseits erhöhte Kosten bei deren Gewinnung aus dem , Fermentationsmedium verursacht und die Raum-Zeit-Ausbeute abnimmt. Diese Erwägungen sind insbesondere für den Fall maßgeblich, wenn ein für eine großvolumige Bioethanol-Produktion hergestelltes Stärkehydrolysat, das herkömmlicherweise geringe Zucker- bzw. Glucosekonzentrationen von bis zu etwa 30 oder 33 Gew.-% aufweist, teilweise einer geringervolumigen Nebenfermentation zur Herstellung anderer Chemikalien zugeführt werden soll.

Aufgrund der dargestellten Schwierigkeiten und Einschränkungen sind Dry-Milling-Verfahren, wie sie für die Bioethanolherstellung in breitem Umfang eingesetzt werden, bei der fermentativen Herstellung anderer mikrobieller Stoffwechselprodukte als Ethanol bislang ohne wesentliche wirtschaftliche Bedeutung geblieben.

Versuche zur Übertragung des Dry-Milling-Konzepts und der mit diesem Verfahren verbundenen prinzipiellen Vorteile auf die großtechnische Herstellung mikrobieller Stoffwechselprodukte sind bisher lediglich unter Verwendung von Cassava als Stärkequelle beschrieben worden. So beschreibt die JP 2001/275693 ein Verfahren zur fermentativen Herstellung von Aminosäuren, bei dem man als Stärkequelle geschälte Cassavaknollen einsetzt, die trocken vermahlen wurden. Zur Durchführung des Verfahrens ist es jedoch erforderlich, eine Teilchengröße des Mahlgutes von ≤ 150 µm einzustellen. Bei der dazu angewendeten Filtration werden Teile des eingesetzten Mahlguts, einschließlich nicht-stärkehaltiger Bestandteile, vor der Verflüssigung/Verzuckerung der enthaltenen Stärke und der anschließenden Fermentation abgetrennt. Bei diesem Verfahren werden mäßige Zuckerkonzentrationen erreicht. Ein ähnliches Verfahren wird in der JP 2001/309751 zur Herstellung eines aminosäurehaltigen Futterzusatzes beschrieben.

Erhöhte Zuckerkonzentrationen in dem zur Fermentation eingesetzten Flüssigmedium können bei Einsatz eines Mahlguts zur Verzuckerung, das weitgehend die festen, nicht-stärkehaltigen Bestandteile der Stärkequelle enthält, durch das in der WO 2005/116228 (PCT/EP2005/005728) der Anmelderin beschriebene Verfahren erreicht werden. Eine Abtrennung der in der Stärkequelle enthaltenen festen, nichtstärkehaltigen Bestandteile vor der Fermentation hat sich dabei überraschenderweise als nicht erforderlich erwiesen. Ein ähnliches Verfahren unter Einsatz von unter Getreidekörnern ausgewählten Stärkequellen wird in der PCT/EP2006/066057 (ältere Patentanmeldung DE 102005042541.0) der Anmelderin beschrieben. In einigen Fällen wurde eine Hemmung oder Verzögerung der Vermehrung der Mikroorganismen beobachtet.

Es war daher Aufgabe der vorliegenden Erfindung, ein weiteres Verfahren zur Herstellung von Lysin durch Fermentation bereitzustellen, das keine oder zumindest keine vollständige vorherige Abtrennung der in der Stärkequelle enthaltenen, nicht-stärkehaltigen festen Bestandteile erfordert. Außerdem sollte es sich durch eine leichte Handhabbarkeit der verwendeten Medien und deren problemlosen Einsatz in der Fermentation auszeichnen. Insbesondere sollte das Verfahren die Verwendung von Getreide als Stärkequelle erlauben.

Es wurde überraschend gefunden, dass sich die hier geschilderten Probleme des Standes der Technik dadurch überwinden lassen, dass man zunächst durch Vermahlen, Verflüssigen und Verzuckern von Stärkequellen ohne vorheriges Abtrennen der nicht-stärkehaltigen festen Bestandteile der Stärkequelle ein erstes zuckerhaltiges Flüssigmedium (1) herstellt und dieses zusammen mit verstoffwechselbaren Mono-, Dioder Oligosacchariden oder einer Zusammensetzung, welche verstoffwechselbare Mono- oder Oligosaccharide in einer Konzentration von wenigstens 50 Gew.-% enthält und die im Wesentlichen frei ist von in Wasser unlöslichen Feststoffen, einer Fermentation zuführt.

Gegenstand der Erfindung ist somit ein Verfahren zur Herstellung von Lysin durch Fermentation, umfassend die folgenden Schritte:
a1) Vermahlen von Getreidekörnern als Stärkequelle unter Erhalt eines Mahlguts, das wenigstens 50 Gew.-% der in den vermahlenen Getreidekörnern enthaltenen nicht-stärkehaltigen festen Bestandteile enthält, wobei der Anteil der nicht-stärkehaltigen festen Bestandteile im Mahlgut wenigstens 15 Gew.-% beträgt;
a2) Suspendieren des Mahlguts in einer wässrigen Flüssigkeit und Hydrolyse des Stärkeanteils im Mahlgut durch enzymatisches Verflüssigen und gegebenenfalls anschließendes Verzuckern, wobei man eine erste Mono- oder Oligosaccharide enthaltende Flüssigkeit (1) erhält, welche die nicht-stärkehaltigen festen Bestandteile des Mahlguts enthält und worin die Gesamtkonzentration an Mono-, Di- und Oligosacchariden im Bereich von 100 bis 400 g/kg liegt; und
b) Zugabe Mono- oder Oligosaccharide enthaltener Flüssigkeit (1) zusammen mit verstoffwechselbaren Mono-, Di- und/oder Oligosacchariden oder einer Zusammensetzung, die verstoffwechselbare Mono-, Di- und und/oder Oligosaccharide in einer Konzentration von wenigstens 50 Gew.-% enthält und die im Wesentlichen frei ist von in Wasser unlöslichen Feststoffen, zu einem Fermentationsmedium, das einen Mikroorganismus enthält, welcher zur Überproduktion des Lysins befähigt ist, unter Fermentationsbedingungen,
wobei man die Gesamtkonzentration an Mono-, Di- und Oligosacchariden in der ersten Flüssigkeit (1) durch Zugabe von verstoffwechselbaren Mono-, Diund/oder Oligosacchariden oder durch Zugabe von einem Medium, das verstoffwechselbare Mono-, Di- und/oder Oligosaccharide in einer Konzentration von wenigstens 50 Gew.-% enthält und das im Wesentlichen frei ist von in Wasser unlöslichen Feststoffen, um wenigstens 50 g/kg erhöht.

Der Einsatz der durch enzymatische Hydrolyse erhaltenen, Mono- oder Oligosaccharide enthaltenen Flüssigkeit (1) führt zu einer deutlichen Senkung der Kosten bei der fermentativen Herstellung der organischen Verbindungen. Aufgrund der hierzu parallelen Zugabe an verstoffwechselbaren Zuckern (d. h. den verstoffwechselbaren Mono-, Di- oder Oligosacchariden) in konzentrierter Form wird zudem eine unerwünschte Verdünnung der Fermentationsbrühe vermieden. Zudem können durch das erfindungsgemäße Verfahren Viskositätsprobleme, wie sie beim Verflüssigen der Stärkequelle bei höheren Konzentrationen an Mahlgut auftreten können, vermieden werden. Außerdem können Probleme bei der Vermehrung des Mikroorganismus auf diese Weise vermieden werden.

Hier und im Folgenden werden in Bezug auf die Mono- oder Oligosaccharide enthaltende Flüssigkeit (1) die Begriffe "Flüssigkeit (1)" und Flüssigmedium (1)" synonym verwendet.

Als Stärkequelle dienen für das erfindungsgemäße Verfahren Getreidekörner, die in getrocknetem Zustand mindestens 40 Gew.-% und bevorzugt mindestens 50 Gew.-% Stärkeanteil aufweisen. Diese finden sich in vielen der heutzutage in großem Maßstab kultivierten Getreidepflanzen wie Mais, Weizen, Hafer, Gerste, Roggen, Triticale, Reis und verschiedenen Hirsesorten, z. B. Sorghum und Milo. Bevorzugt ist die Stärkequelle unter Mais-, Roggen-, Triticale- und Weizenkörnern ausgewählt. Grundsätzlich lässt sich das erfindungsgemäße Verfahren auch mit analogen Stärkequellen durchführen, wie beispielsweise einer Mischung verschiedener stärkehaltiger Getreidekörner.

Zur Herstellung des Flüssigmediums (1) wird im Schritt a1) die jeweilige Stärkequelle mit oder ohne Zusatz von Flüssigkeit, z. B. Wasser, vermahlen, bevorzugt ohne Zusatz von Flüssigkeit. Es kann auch eine Trockenvermahlung mit einer anschließenden Nassvermahlung kombiniert werden.

Zur trockenen Vermahlung werden typischerweise Hammermühlen, Rotormühlen oder Walzen-Schrotmühlen eingesetzt; zur Nassvermahlung eignen sich Rührmixer, Rührwerkskugelmühlen, Zirkulationsmühlen, Scheibenmühlen, Ringkammermühlen, Schwingmühlen oder Planetenmühlen. Grundsätzlich kommen auch andere Mühlen in Betracht. Die zur Nassvermahlung erforderliche Flüssigkeitsmenge kann der Fachmann in Routineexperimenten ermitteln. Üblicherweise wird sie so eingestellt, dass der Gehalt an Trockensubstanz im Bereich von 10 bis 20 Gew.-% liegt.

Durch das Mahlen wird eine für die sich anschließenden Verfahrensschritte geeignete Korngröße eingestellt. Hierbei hat es sich als vorteilhaft erwiesen, wenn das beim Vermahlen, insbesondere bei trockener Vermahlung, im Schritt a1) erhaltene Mahlgut Mehlpartikel, d. h. teilchenförmige Bestandteile, mit einer Korngröße im Bereich von 100 bis 630 µm in einem Anteil von 30 bis 100 Gew.-%, bevorzugt 40 bis 95 Gew.-% und besonders bevorzugt 50 bis 90 Gew.-% aufweist. Vorzugsweise enthält das erhaltene Mahlgut 50 Gew.-% an Mehlpartikeln mit einer Korngröße von mehr als 100 µm. In der Regel weisen mindestens 95 Gew.-% der vermahlenen Mehlpartikel eine Korngröße von weniger als 2 mm auf. Die Messung der Korngröße erfolgt dabei mittels Siebanalyse unter Verwendung einer Vibrations-Analysenmaschine. Eine geringe Korngröße ist grundsätzlich zur Erzielung einer hohen Produktausbeute vorteilhaft. Eine zu geringe Teilchengröße kann jedoch zu Problemen, insbesondere durch Klumpenbildung/Agglomeration, beim Anmaischen des Mahlguts während der Verflüssigung bzw. bei der Aufarbeitung, z. B. bei der Trocknung der Feststoffe nach dem Fermentationsschritt, führen.

Üblicherweise werden Mehle durch den Ausmahlungsgrad bzw. durch die Mehltype charakterisiert, wobei diese so miteinander korrelieren, dass mit zunehmendem Ausmahlungsgrad auch die Kennzahl der Mehltype zunimmt. Der Ausmahlungsgrad entspricht der Gewichtsmenge des gewonnenen Mehls, bezogen auf 100 Gewichtsteile des eingesetzten Mahlguts. Während beim Mahlen zunächst reines, feinstes Mehl, z. B. aus dem Inneren des Getreidekorns, anfällt, nimmt beim weiteren Vermahlen, also mit steigendem Ausmahlungsgrad, der Anteil an Rohfaser- und Schalengehalt im Mehl zu, der Stärkeanteil wird dabei geringer. Der Ausmahlungsgrad spiegelt sich daher auch in der sogenannten Mehltype wider, die als Zahlenangabe zur Klassifizierung von Getreidemehlen verwendet wird und die auf dem Aschegehalt des Mehles beruht (sogenannte Ascheskala). Die Mehltype bzw. die Typenzahl gibt hierbei die Menge Asche (Mineralstoffe) in mg an, die beim Verbrennen von 100 g Mehltrockensubstanz zurück bleibt. Für Getreidemehle bedeutet eine höhere Typzahl einen höheren Ausmahlungsgrad, da der Kern des Getreidekorns in etwa 0,4 Gew.-%, die Schale hingegen etwa 5 Gew.-% Asche enthält. Bei niederem Ausmahlungsgrad bestehen die Getreidemehle also überwiegend aus dem zerkleinerten Mehlkörper, d. h. dem Stärkebestandteil der Getreidekörner; bei höherem Ausmahlungsgrad enthalten die Getreidemehle auch die zerkleinerte, eiweißhaltige Aleuronschicht der Getreidekörner, bei Schrot auch die Bestandteile des eiweiß- und fetthaltigen Keimlings sowie der rohfaser- und aschehaltigen Samenschalen. Für die erfindungsgemäßen Zwecke sind grundsätzlich Mehle mit einem hohen Ausmahlungsgrad bzw. einer hohen Typzahl bevorzugt. Vorzugsweise werden die ganzen ungeschälten Körner vermahlen und weiter verarbeitet, gegebenenfalls nach vorheriger mechanischer Abtrennung von Keim und Spelzen.

Erfindungsgemäß enthält das zur Herstellung des Flüssigmediums (1) verwendete Mahlgut wenigstens 50 Gew.-%, speziell wenigstens 90 Gew.-% und ganz speziell wenigstens 99 Gew.-% der in den vermahlenen Getreidekörnern enthaltenen nicht-stärkehaltigen festen Bestandteile, entsprechend dem Ausmahlungsgrad. Bezogen auf die stärkehaltigen Bestandteile des Mahlguts (und damit auf die Menge an Mono-, Di- oder Oligosaccharid im Flüssigmedium (1)) beträgt der Anteil der nicht-stärkehaltigen festen Bestandteile im Mahlgut wenigstens 15 Gew.-%, z. B. von 15 bis 75 Gew.-% und speziell im Bereich von 20 bis 60 Gew.-%.

Die enzymatische Hydrolyse des Mahlguts gemäß Schritt a2) kann nach üblichen, dem Fachmann bekannten Verfahren erfolgen, z. B. nach den in dem eingangs zitierten "The Alcohol Textbook - A reference for the beverage, fuel and industrial alcohol industries", Kapitel 2, S. 7 bis 23 beschriebenen Methoden.

Hierzu wird man zunächst das Mahlgut mit einer wässrigen Flüssigkeit, z. B. Frischwasser, rückgeführtem Prozesswasser, z. B. aus einer nachfolgenden Fermentation, oder mit einer Mischung dieser Flüssigkeiten vermischen, wobei man eine wässrige Suspension erhält. Dieser Vorgang wird häufig auch als Anmaischen bezeichnet.

Die Menge an Mahlgut und wässriger Flüssigkeit werden in der Regel so gewählt, dass man durch die Hydrolyse ein wässriges Flüssigmedium (1) erhält, worin die Gesamtkonzentration an Mono- Di-, und/oder Oligosacchariden im Bereich von 100 bis 400 g/kg, vorzugsweise im Bereich von 150 bis 350 g/kg und insbesondere im Bereich von 200 bis 300 g/kg liegt. Dementsprechend setzt man das Mahlgut typischerweise in einer Menge von 150 bis 550 g/kg, häufig im Bereich von 200 bis 500 g/kg und insbesondere im Bereich von 250 bis 450 g/kg, bezogen auf das Gesamtgewicht der Suspension (Maische), ein. Man kann grundsätzlich auch größere Mengen an Mahlgut einsetzen, um höhere Gesamtkonzentration an Mono- Di-, und/oder Oligosacchariden zu erreichen, z. B. Konzentrationen oberhalb 400 g/kg bis 700 g/kg, insbesondere im Bereich von 450 bis 650 g/kg oder 500 g/kg bis 650 g/kg zu erreichen.

Zur enzymatischen Hydrolyse des Stärkeanteils des Mahlguts wird man in der Regel zuerst eine Verflüssigung des Mahlguts in Gegenwart eines die Stärke verflüssigenden Enzyms, in der Regel eine α-Amylase, vornehmen. Andere unter den Reaktionsbedingungen aktive und stabile Stärke verflüssigende Enzyme sind ebenfalls einsetzbar.

Zur Verflüssigung des Stärkeanteils im Mahlgut können grundsätzlich alle verflüssigenden Enzyme, insbesondere α-Amylasen (Enzymklasse EC 3.2.1.1) eingesetzt werden, beispielsweise α-Amylasen, die aus *Bacillus lichenformis* oder *Bacillus staerothermophilus* gewonnen wurden und speziell solche, die zum Verflüssigen von durch Dry-Milling-Verfahren gewonnenen Materialien im Rahmen der Herstellung von Bioethanol verwendet werden. Die zum Verflüssigen geeigneten α-Amylasen sind auch kommerziell erhältlich, beispielsweise von Novozymes unter der Bezeichnung Termamyl 120 L, Typ L; oder von Genencor unter der Bezeichnung Spezyme. Es kann auch eine Kombination verschiedener α-Amylasen zur Verflüssigung eingesetzt werden.

Hierbei erhält man ein wässriges Medium, welches den verflüssigten Stärkeanteil aus dem Mahlgut, typischerweise Oligosaccharide mit in der Regel 3 bis 18, insbesondere 6 bis 12 Monosaccharid-Einheiten, insbesondere Glucoseeinheiten, sowie die nicht-stärkehaltigen Bestandteile des eingesetzten Mahlguts, insbesondere die festen, nicht-stärkehaltigen Bestandteile des zur Verflüssigung eingesetzten Mahlguts enthält.

Vorteilhafterweise werden die Mengen an Stärke verflüssigendem Enzym und Mahlgut, so gewählt sein, dass die Viskosität während des Gelierungsvorgangs ausreichend reduziert ist, um eine effektive Durchmischung der Suspension, z. B. mittels Rühren, zu ermöglichen. Bevorzugt beträgt die Viskosität der Reaktionsmischung während des Gelierens maximal 20 Pas, besonders bevorzugt maximal 15 Pas und ganz besonders bevorzugt maximal 8 Pas. Die Messung der Viskosität erfolgt in der Regel mit einem Haake Viskosimeter Type Roto Visko RV20 mit Messsystem M5 und Messeinrichtung MVDIN bei einer Temperatur von 50 °C und einer Schergeschwindigkeit von 200 s⁻¹.

Die α-Amylase (bzw. das verwendete Stärke verflüssigende Enzym) kann im Reaktionsgefäß vorgelegt oder im Verlauf des Schrittes a2) zugegeben werden. Vorzugsweise gibt man eine Teilmenge der in Schritt a2) benötigten α-Amylase zu Beginn von Schritt a2) zu oder legt diese Teilmenge im Reaktor vor. Die Gesamtmenge an α-Amylase liegt üblicherweise im Bereich von 0,002 bis 3,0 Gew.-%, bevorzugt von 0,01 bis 1,5 Gew.-% und besonders bevorzugt von 0,02 bis 0,5 Gew.-%, bezogen auf die Gesamtmenge der eingesetzten Stärkequelle.

Die Verflüssigung kann oberhalb oder unterhalb der Gelierungstemperatur durchgeführt werden. Vorzugsweise erfolgt die Verflüssigung in Schritt a2) zumindest zeitweise oberhalb der Gelierungstemperatur bzw. Verkleisterungstemperatur der eingesetzten Stärke (so genannter Cooking Prozess). Die hierzu für die jeweilige Stärke erforderliche Temperatur ist dem Fachmann bekannt (siehe das eingangs zitierte "The Alcohol Textbook - A reference for the beverage, fuel and industrial alcohol industries", Kapitel 2, S. 11) oder kann von ihm in Routineexperimenten bestimmt werden. In der Regel wird eine Temperatur im Bereich zwischen 80 und 165 °C, bevorzugt zwischen 90 und 150 °C und besonders bevorzugt im Bereich von 100 bis 140 °C gewählt, wobei die Temperatur in der Regel mindestens 5 K, insbesondere mindestens 10 K und besonders bevorzugt mindestens 20 K, z. B. 10 bis 100 K, insbesondere 20 bis 80 K oberhalb der Gelierungstemperatur liegt. Bei diesen Temperaturen wird die granuläre Struktur der Stärke zerstört (Gelieren), wodurch deren enzymatischer Abbau ermöglicht wird.

Für eine optimale Wirkung der α-Amylase (bzw. des verwendeten Stärke verflüssigenden Enzyms) wird Schritt a2) vorzugsweise zumindest zeitweise im pH-Optimum des verflüssigenden Enzyms, häufig bei einem pH-Wert im schwäch sauren Bereich, vorzugsweise zwischen 4,0 und 7,0, besonders bevorzugt zwischen 5,0 bis 6,5 durchgeführt, wobei üblicherweise vor oder zu Beginn von Schritt a2) die pH-Einstellung vorgenommen wird; dieser pH-Wert wird während der Verflüssigung vorzugsweise kontrolliert und gegebenenfalls nachgestellt. Die Einstellung des pH-Werts erfolgt vorzugsweise mit verdünnten Mineralsäuren wie H₂SO₄ oder H₃PO₄ bzw. mit verdünnten Alkalilaugen wie NaOH oder KOH.

In einer bevorzugten Ausführungsform gibt man zum Verflüssigen des Stärkeanteils im Mahlgut gemäß Schritt a2) mindestens eine Teilmenge des Mahlguts kontinuierlich oder diskontinuierlich zu der wässrigen Flüssigkeit. Vorzugsweise gibt man hierbei wenigstens 40 Gew.-%, insbesondere wenigstens 50 Gew.-% und ganz besonders bevorzugt wenigstens 55 Gew.-% im Verlauf der Verflüssigung, jedoch vor einer etwaigen Verzuckerung in den Reaktor. Häufig wird die zugegebene Menge 90 Gew.-%, insbesondere 85 Gew.-% und besonders bevorzugt 80 Gew.-% nicht überschreiten. Vorzugsweise wird die im Verlauf zugegebene Teilmenge an Mahlgut dem Reaktor unter Bedingungen zugeführt, wie sie bei der Verflüssigung vorliegen. Die Zugabe kann diskontinuierlich, d. h. portionsweise in mehreren Teilportionen, die vorzugsweise jeweils nicht mehr als 30 Gew.-%, besonders bevorzugt nicht mehr als 20 Gew.-%, z. B. 1 bis 30 Gew.-% und insbesondere 2 bis 20 Gew.-%, der Gesamtmenge des zu verflüssigenden Mahlgutes ausmachen, oder kontinuierlich erfolgen. Wesentlich bei dieser Ausführungsform ist, dass sich zu Beginn der Verflüssigung nur ein Teil des Mahlgutes, vorzugsweise nicht mehr als 60 Gew.-%, insbesondere nicht mehr als 50 Gew.-% und besonders bevorzugt nicht mehr als 45 Gew.-% des Mahlgutes, im Reaktor befindet und die Restmenge des Mahlgutes während des Verflüssigens zugegeben wird.

Die Verflüssigung kann auch kontinuierlich, z. B. in einer mehrstufigen Reaktionskaskade, durchgeführt werden.

In einer bevorzugten Ausführungsform führt man Schritt a2) des erfindungsgemäßen Verfahrens so durch, dass zunächst eine Teilmenge von höchstens 60 Gew.-%, bevorzugt höchstens 50 Gew.-% und besonders bevorzugt höchstens 45 Gew.-%, z: B. 10 bis 60 Gew.-%, insbesondere 15 bis 50 Gew.-% und besonders bevorzugt 20 bis 45 Gew.-%, bezogen auf die Gesamtmenge des Mahlguts, in der wässrigen Flüssigkeit, suspendiert wird und anschließend die Verflüssigung durchgeführt wird.

In einer bevorzugten Ausführungsform erfolgt die diskontinuierliche oder kontinuierliche, insbesondere portionsweise Zugabe einer Teilmenge des Mahlguts in Schritt a2) derart, dass die Viskosität des Flüssigmediums maximal 20 Pas, bevorzugt maximal 15 Pas und besonders bevorzugt maximal 8 Pas beträgt. Zur Unterstützung der Viskositätskontrolle hat es sich als vorteilhaft erwiesen, wenn mindestens 25 Gew.-%, bevorzugt mindestens 35 Gew.-% und besonders bevorzugt mindestens 50 Gew.-% der Gesamtmenge des zugegebenen Mahlguts bei einer Temperatur oberhalb der Gelatinierungstemperatur der im Mahlgut enthaltenen Stärke zugegeben werden. Die Kontrolle der Viskosität kann des Weiteren dadurch beeinflusst werden, dass das mindestens eine Stärke verflüssigende Enzym, vorzugsweise eine α-Amylase, oder/und das mindestens eine verzuckernde Enzym, vorzugsweise eine Glucoamylase, selbst portionsweise zugegeben werden.

Zur Durchführung des erfindungsgemäßen Verfahrens ist es möglich, die zum Suspendieren des festen Mahlguts verwendete wässrige Flüssigkeit auf eine leicht erhöhte Temperatur, z. B. im Bereich von 40 bis 60 °C, vorzutemperieren. Es ist jedoch bevorzugt, die Flüssigkeiten bei Raumtemperatur einzusetzen.

Zu der Suspension des Mahlguts wird dann das mindestens eine Stärke verflüssigende Enzym, vorzugsweise eine α-Amylase, gegeben. Wird eine Teilmenge des Mahlguts erst im Verlauf der Verflüssigung hinzugegeben, so gibt man anfangs vorteilhafterweise nur eine Teilmenge der α-Amylase zu, z.B. 10 bis 70 Gew.-% und insbesondere 20 bis 65 Gew.-%, bezogen auf die insgesamt in Schritt a2) eingesetzte α-Amylase. Die zu diesem Zeitpunkt zugegebene Menge an α-Amylase richtet sich in diesem Fall nach der Aktivität der jeweiligen α-Amylase in Bezug auf die verwendete Stärkequelle unter den Reaktionsbedingungen und liegt üblicherweise im Bereich von 0,0004 bis 2,0 Gew.-%, bevorzugt von 0,001 bis 1,0 Gew.-% und besonders bevorzugt von 0,02 bis 0,3 Gew.-%, bezogen auf die Gesamtmenge der eingesetzten Stärkequelle. Alternativ kann hierbei die Teilmenge der α-Amylase vor dem Ansetzen der Suspension mit der verwendeten Flüssigkeit vermengt werden.

Vorzugsweise wird die eingesetzte Menge oder Teilmenge an α-Amylase vor dem Beginn des Aufheizens auf die zur Verflüssigung angewendete Temperatur, insbesondere bei Raumtemperatur oder nur leicht erhöhter Temperatur, z. B. im Bereich von 20 bis 30 °C, zu der Suspension gegeben.

Die so angesetzte Suspension wird dann vorzugsweise auf eine Temperatur oberhalb der Gelierungstemperatur der verwendeten Stärke erhitzt. In der Regel wird eine Temperatur im Bereich von 80 bis 165 °C, bevorzugt von 90 bis 150 °C und besonders bevorzugt im Bereich von 100 bis 140 °C gewählt, wobei die Temperatur vorzugsweise mindestens 5 K, insbesondere 10 K und besonders bevorzugt mindestens 20 K, z. B. 10 bis 100 K, insbesondere 20 bis 80 K, oberhalb der Gelierungstemperatur (Verkleisterungstemperatur) liegt. Unter Kontrolle der Viskosität werden gegebenenfalls nach und nach weitere Teilmengen der Stärkequelle, z. B. jeweils 1 bis 30 Gew.-% und insbesondere 2 bis 20 Gew.-%, bezogen auf die gesamte eingesetzte Menge an Mahlgut, zu der stärkehaltigen Suspension gegeben. In diesem Fall ist es bevorzugt, die im Verlauf der Verflüssigung zuzugebende Teilmenge des Mahlgutes in mindestens 2, bevorzugt mindestens 4 und besonders bevorzugt mindestens 6 Teilportionen der Reaktionsmischung zuzugeben. Alternativ kann in dieser Ausführungsform die Zugabe der beim Ansetzen der Suspension nicht eingesetzten Teilmenge des Mahlgutes kontinuierlich während der Verflüssigung erfolgen. Die Temperatur sollte bei der Zugabe vorteilhafterweise oberhalb der Gelierungstemperatur der Stärke gehalten werden.

Nach Erreichen der gewünschten Temperatur bzw. gegebenenfalls nach vollständiger Zugabe des Mehls wird das Reaktionsgemisch üblicherweise noch eine Zeit, z. B. 10 bis 60 Minuten oder länger, sofern erforderlich, bei der oberhalb der Gelierungstemperatur der Stärke eingestellten Temperatur gehalten, d. h. verkocht. Die Reaktionsmischung wird dann in der Regel auf eine etwas geringere Temperatur, jedoch vorzugsweise oberhalb der Gelierungstemperatur, z. B. auf 70 bis 90 °C, abgekühlt. Anschließend wird gegebenenfalls eine weitere Teilmenge an α-Amylase, vorzugsweise die Hauptmenge, zugesetzt. In diesem Fall beträgt die Menge an zu diesem Zeitpunkt zugegebener α-Amylase je nach Aktivität der verwendeten α-Amylase unter den Reaktionsbedingungen vorzugsweise 0,002 bis 2,0 Gew.-%, besonders bevorzugt von 0,01 bis 1,0 Gew.-% und ganz besonders bevorzugt von 0,02 bis 0,4 Gew.-%, bezogen auf die Gesamtmenge der eingesetzten Stärkequelle.

Zum vollständigen Abbau der Stärke zu Dextrinen wird das Reaktionsgemisch so lange bei der eingestellten Temperatur gehalten oder gegebenenfalls weiter erhitzt, bis der Stärkenachweis mit Jod oder gegebenenfalls ein anderer Test zum Nachweis von Stärke negativ oder mindestens im Wesentlichen negativ ausfällt. Gegebenenfalls können hierbei noch eine oder mehrere weitere Teilmengen α-Amylase, z. B. im Bereich von 0,001 bis 0,5 Gew.-% und bevorzugt 0,002 bis 0,2 Gew.-%, bezogen auf die Gesamtmenge der eingesetzten Stärkequelle, zu der Reaktionsmischung gegeben werden.

Alternativ kann man die das Mahlgut enthaltende wässrige Suspension zur Verflüssigung des Stärkeanteils zunächst durch Einbringen von Wasserdampf auf eine Temperatur oberhalb der Verkleisterungstemperatur der in der Stärkequelle bzw. dem Mahlgut enthaltenen Stärke erhitzen. Typischerweise wird man auf eine Temperatur erhitzen, die wenigstens 10 K und insbesondere wenigstens 20 K, z. B. 10 bis 100 K, insbesondere 20 bis 80 K oberhalb der jeweiligen Verkleisterungstemperatur liegt. Insbesondere erhitzt man die Suspension auf Temperaturen im Bereich von 90 bis 150 °C, und speziell im Bereich von 100 bis 140 °C.

Bei dem zum Erhitzen eingesetzten Wasserdampf handelt es sich typischerweise um überhitzen Wasserdampf, der eine Temperatur von wenigstens 105 °C, insbesondere wenigstens 110 °C, z. B. 110 bis 210 °C, aufweist. Vorzugsweise wird der Dampf mit Überdruck in die Suspension eingebracht. Dementsprechend weist der Dampf vorzugsweise einen Druck von wenigstens 1,5 bar, z. B. 1,5 bis 16 bar, insbesondere 2 bis 12 bar auf.

Das Einbringen von Wasserdampf in die Suspension erfolgt in der Regel so, dass man den Dampf mit Überdruck, vorzugsweise einem Überdruck von 1 bis 10 oder 11 bar, insbesondere 1,5 bis 5 bar und vorzugsweise mit hoher Geschwindigkeit in die Suspension einträgt. Durch das Eintragen des Dampfes erhitzt sich die Suspension augenblicklich auf Temperaturen oberhalb 90 °C, also auf Temperaturen oberhalb der Verkleisterungstemperatur.

Vorzugsweise erfolgt das Erhitzen mit Wasserdampf in einer kontinuierlich arbeitenden Vorrichtung, in welche man die Suspension kontinuierlich mit einem bestimmten Förderdruck, der sich aus der Viskosität der Suspension, der Fördergeschwindigkeit und der Geometrie der Vorrichtung ergibt, einspeist und in die man im Bereich des Einspeisens der Suspension den heißen Dampf mit Überdruck, bezogen auf den Förderdruck, über eine regelbare Düse einspeist. Durch das Einspeisen des Dampfs mit Überdruck wird die Suspension nicht nur erhitzt sondern es wird auch mechanische Energie in das System eingetragen, welche eine weitere Zerteilung der Mahlgutpartikel fördert, einen besonders gleichmäßigen Energieeintrag bewirkt, und somit eine besonders gleichmäßige Verkleisterung der granulären Stärkepartikel im Mahlgut zur Folge hat. Typischerweise haben diese Vorrichtungen eine röhrenförmige Geometrie. Vorzugsweise erfolgt das Eintragen des Dampfes in Richtung der Längsachse der röhrenförmigen Vorrichtung. Die Zufuhr der Suspension erfolgt in der Regel in einem Winkel von wenigstens 45° oder senkrecht hierzu. Die regelbare Düse weist typischerweise eine konische Geometrie auf, die sich in Fließrichtung des Dampfs verjüngt. In dieser Düse ist eine Nadel oder ein auf einer in Längsrichtung verschiebbaren Stange angeordneter Kegel angeordnet. Nadel bzw. Kegel bildet mit dem Konus der Düse einen Spalt. Durch Verschieben der Nadel bzw. der Stange in Längsrichtung lässt sich die Größe des Spalts und damit die Querschnittsfläche der Düsenöffnung in einfacher Weise einstellen, wodurch man in einfacher Weise die Geschwindigkeit des Dampfeintrags regulieren kann.

Typischerweise weisen diese Vorrichtungen außerdem ein Vermischungsrohr auf, in welches die Suspension nach dem Dampfeintrag transportiert und aus der Vorrichtung ausgetragen wird. Dieses Vermischungsrohr ist üblicherweise in Richtung des Dampfeintrags und senkrecht zur Einspeisung angeordnet. Das Vermischungsrohr bildet typischerweise mit der Düse einen Spalt, durch den die Suspension transportiert wird. Durch diesen Spalt wirken beim Transport zusätzliche Scherkräfte auf die Suspension und erhöhen somit den mechanischen Energieeintrag in die Suspension. Das Vermischungsrohr kann in Längsrichtung verschiebbar angeordnet sein. Durch Verschieben des Vermischungsrohrs lässt sich in einfacher Weise die Größe der Spaltöffnung einstellen und damit das Druckgefälle in der Vorrichtung.

Derartige Vorrichtungen sind unter der Bezeichnung Jet-Kocher aus dem Stand der Technik bekannt, beispielsweise die in "The Alcohol Textbook", Kapitel 2, loc. cit., Figur 13 dargestellte Vorrichtung und kommerziell erhältlich, beispielsweise unter der Bezeichnung HYDROHEATER® der Fa. Hydro Thermal Corp. Waukesha WI, USA.

Bei kontinuierlicher Reaktionsführung wird die mit Wasserdampf behandelte Suspension in der Regel im Anschluss daran in eine Nachreaktionszone überführt, um das Gelieren der Stärkebestandteile fortzusetzen. In der Nachreaktionszone herrscht typischerweise Überdruck, typischerweise ein Absolutdruck im Bereich von 2 bis 8 bar. Die Temperaturen in der Nachreaktionszone liegen typischerweise im Bereich von 90 bis 150 °C. Die Verweilzeit in dieser Nachreaktionszone kann in Abhängigkeit von der Temperatur der Suspension im Bereich von 1 min bis 4 h betragen. Die Nachreaktionszonen weisen typischerweise eine röhrenförmige oder säulenförmige Geometrie auf. In einer Ausführungsform weist die Nachreaktionszone die Geometrie einer senkrecht angeordneten Säule auf. Die Suspension wird hierbei nach Verlassen der Vorrichtung zur Dampfbehandlung im oberen Bereich der Säule aufgebracht und im unteren Bereich entnommen. In einer anderen Ausführungsform weist die Nachreaktionszone eine röhrenförmige Geometrie auf.

Nach Verlassen der Nachreaktionszone wird die Suspension in der Regel entspannt und man führt dann eine Verflüssigung durch. Vorzugsweise führt man die Entspannung als Flash-Verdampfung durch, um die Suspension abzukühlen, vorzugsweise auf Temperaturen unterhalb 100 °C, insbesondere unterhalb 85 °C. In der Regel erfolgt dann eine Verflüssigung der so aufgeschlossenen Stärke in einem separaten Reaktionsgefäß. Die Verflüssigung kann in der oben beschriebenen Weise durchgeführt werden.

In einer bevorzugten Ausführungsform gibt man wenigstens einen Teil oder die Gesamtmenge, in der Regel wenigstens 50 %, insbesondere wenigstens 80 % der Gesamtmenge oder die Gesamtmenge des die Stärke verflüssigenden Enzyms vor dem Erhitzen mit Wasserdampf zu der Suspension des Mahlguts in der wässrigen Flüssigkeit. Auf diese Weise erfolgt die Verflüssigung bereits während des Erhitzens auf Temperaturen oberhalb der Verkleisterungstemperatur. Das Erhitzen mit Wasserdampf und die Nachreaktionsphase werden entsprechend durchgeführt. Eine anschließende Verflüssigung in einem separaten Reaktionsgefäß kann entfallen. Vorzugsweise wird man jedoch eine solche Verflüssigung zur Vervollständigung des Abbaus der Stärke in Dextrine durchführen.

Zur Stabilisierung der eingesetzten Enzyme kann gegebenenfalls die Konzentration an Ca²⁺-Ionen, z. B. mit CaCl₂, auf einen enzymspezifischen optimalen Wert eingestellt werden. Geeignete Konzentrationswerte können vom Fachmann in Routineexperimenten bestimmt werden. Wird z. B. Termamyl als α-Amylase eingesetzt, so ist es vorteilhaft, eine Ca²⁺-Konzentration von z. B. 10 bis 100 ppm, bevorzugt 20 bis 80 ppm und besonders bevorzugt etwa 30 bis 70 ppm im Flüssigmedium einzustellen, wobei die Angabe ppm gewichtsbezogen ist und g/1000 kg bedeutet.

Zum vollständigen Abbau der Stärke zu Dextrinen wird das Reaktionsgemisch so lange bei der eingestellten Temperatur gehalten, bis der Stärkenachweis mit Jod oder gegebenenfalls ein anderer Test zum Nachweis von Stärke negativ oder mindestens im Wesentlichen negativ ausfällt. Gegebenenfalls können hierbei noch eine oder mehrere weitere Teilmengen α-Amylase, z. B. im Bereich von 0,001 bis 0,5 Gew.-% und bevorzugt 0,002 bis 0,2 Gew.-%, bezogen auf die Gesamtmenge der eingesetzten Stärkequelle, zu der Reaktionsmischung gegeben werden.

Auf diese Weise erhält man eine wässriges Stärkehydrolysat, welches den verflüssigten Stärkeanteil aus dem Mahlgut, typischerweise Dextrine und gegebenenfalls weitere Oligosaccharide und Mono- oder Disaccharide, sowie die nicht-stärkehaltigen Bestandteile des Mahlguts, insbesondere die festen, nicht-stärkehaltigen Bestandteile des zur Verflüssigung eingesetzten Mahlguts enthält.

Nach abgeschlossener Verflüssigung der Stärke kann eine Verzuckerung der in dem Flüssigmedium enthaltenen Dextrine, d. h. deren Abbau zu Glucose, kontinuierlich oder diskontinuierlich in an sich bekannter Weise durchgeführt werden. Das verflüssigte Medium kann in einem speziellen Verzuckerungstank vollständig verzuckert werden, bevor es z. B. einem nachfolgenden Fermentationsschritt zugeführt wird.

In einer ersten Ausführungsform wird vor der nachfolgenden Fermentation nur eine teilweise Verzuckerung durchgeführt. Beispielsweise kann man so vorgehen, dass man eine Teilmenge der in dem Flüssigmedium enthaltenen Dextrine, z. B. im Bereich von 10 bis 90 Gew.-% und insbesondere im Bereich von 20 bis 80 Gew.-%, bezogen auf das Gesamtgewicht der Dextrine (bzw. der ursprünglichen Stärke), verzuckert und das resultierende zuckerhaltige Flüssigmedium in der Fermentation einsetzt. Im Fermentationsmedium kann dann eine weitere Verzuckerung in situ erfolgen. Die Verzuckerung kann des Weiteren unter Wegfall eines separaten Verzuckerungstanks direkt im Fermenter (in situ) durchgeführt werden.

Vorteile der in-situ-Verzuckerung, d. h. einer teilweise oder vollständig im Fermenter erfolgenden Verzuckerung, sind einerseits reduzierte Investitionskosten, andererseits kann durch eine retardierte Freisetzung der Glucose gegebenenfalls eine höhere Glucosekonzentration im Ansatz (Batch) vorgelegt werden, ohne dass eine Inhibierung oder Stoffwechseländerung der eingesetzten Mikroorganismen auftritt. Bei *E.coli* führt eine zu hohe Glucosekonzentration z. B. zur Bildung von organischen Säuren (Acetat), während *Saccharomyces cerevisae* in diesem Fall z. B. auf Vergärung umschaltet, obwohl in belüfteten Fermentern ausreichend Sauerstoff vorhanden ist (Crabtree-Effekt). Eine retardierte Freisetzung von Glucose ist durch die Regelung der Glucoamylasekonzentration einstellbar. Hierdurch können die vorgenannten Effekte unterdrückt werden und es kann mehr Substrat vorgelegt werden, so dass die aus dem zugeführten Feed-Strom resultierende Verdünnung reduziert werden kann.

Zur Verzuckerung der Dextrine (d. h. Oligosaccharide) in der verflüssigten Stärkelösung erfolgt auf enzymatischem Wege, d. h. mit Hilfe wenigstens eines die Dextrine verzuckernden Enzyms. Hierzu können grundsätzlich alle Glucoamylasen (Enzymklasse EC 3.2.1.3) eingesetzt werden, insbesondere Glucoamylasen, die aus *Aspergilus* gewonnen wurden und speziell solche, die zum Verzuckern von durch Dry-Milling-Verfahren gewonnenen Materialien im Rahmen der Herstellung von Bioethanol verwendet werden. Die zum Verzuckern geeigneten Glucoamylasen sind auch kommerziell erhältlich, beispielsweise von Novozymes unter der Bezeichnung Dextrozyme GA; oder von Genencor unter der Bezeichnung Optidex. Es kann auch eine Kombination verschiedener Glucoamylasen verwendet werden.

Das wenigstens eine verzuckernde Enzym, insbesondere wenigstens eine Glucoamylase, wird dem nach der Verflüssigung erhaltenen dextrinhaltigen Flüssigmedium üblicherweise in einer Menge von 0,001 bis 5,0 Gew.-%, bevorzugt von 0,005 bis 3,0 Gew.-% und besonders bevorzugt von 0,01 bis 1,0 Gew.-%, bezogen auf die Gesamtmenge der eingesetzten Stärkequelle, zugesetzt.

Sofern man die Verzuckerung im Fermenter durchführt, wird man die verflüssigte Stärkelösung in der Regel auf Fermentationstemperatur, d. h. 32 bis 37 °C, abkühlen, bevor man sie dem Fermenter zuführt. Die Glucoamylase (bzw. das mindestens eine verzuckernde Enzym) zur Verzuckerung wird in diesem Fall der Fermentationsbrühe direkt zugesetzt. Die Verzuckerung der.verflüssigten Stärke gemäß Schritt a2) erfolgt hierbei parallel zur Verstoffwechselung des Zuckers durch die Mikroorganismen.

Im Fall der Verzuckerung in einem Verzuckerungstankwird die verflüssigte Stärkelösung üblicherweise auf das Temperaturoptimum des verzuckernden Enzyms oder leicht darunter, z. B. auf 50 bis 70 °C, bevorzugt 60 bis 65 °C abgekühlt bzw. temperiert und anschließend mit Glucoamylase versetzt.

Vorteilhafterweise wird vor Zugabe des verzuckernden Enzyms, insbesondere der Glucoamylase, der pH-Wert des Flüssigmediums auf einen Wert im optimalen Wirkungsbereich der eingesetzten Glucoamylase, vorzugsweise im Bereich zwischen 3,5 und 6,0; besonders bevorzugt zwischen 4,0 und 5,5 und ganz besonders bevorzugt zwischen 4,0 und 5,0 eingestellt. Es ist jedoch auch möglich, insbesondere bei Durchführung der Verzuckerung direkt im Fermenter, den pH-Wert außerhalb der vorgenannten Bereiche einzustellen, z. B. im Bereich von größer 6,0 bis 8,0. Dies kann z. B. bei der fermentativen Herstellung von Lysin, Panthothenat und Vitamin B₂ trotz der eingeschränkten Aktivität von Standard-Glucoamylasen in diesem pH Bereich insgesamt vorteilhaft oder aufgrund der einzustellenden Fermentationsbedingungen erforderlich sein.

In einer bevorzugten Ausführungsform erfolgt die Verzuckerung in einem speziellen Verzuckerungstank. Dazu wird die verflüssigte Stärkelösung auf eine für das Enzym optimale Temperatur oder leicht darunter temperiert und der pH-Wert in der oben beschriebenen Weise auf einen für das Enzym optimalen Wert eingestellt.

Nach Zugabe des verzuckernden Enzyms wird die dextrinhaltige Suspension vorzugsweise für einen Zeitraum von z. B. 2 bis 72 Stunden oder länger, sofern erforderlich, insbesondere von 5 bis 48 Stunden bei der eingestellten Temperatur gehalten, wobei die Dextrine zu Monosacchariden verzuckert werden. Der Fortschritt der Verzuckerung kann mit dem Fachmann bekannten Methoden, z. B. HPLC, Enzymtests oder Glucose-Teststäbchen, verfolgt werden. Die Verzuckerung ist abgeschlossen, wenn die Konzentration der Monosaccharide nicht mehr wesentlich ansteigt oder wieder fällt.

Da zur Herstellung des zuckerhaltigen Flüssigmediums (1) in der Regel Mahlgut eingesetzt wird, welches zumindest einen Teil oder alle Bestandteile der Stärkequelle enthält (d. h. eine Abtrennung der nicht-stärkehaltigen festen Bestandteile der Stärkequelle wird nicht oder nicht vollständig vorgenommen), umfasst das erhaltene Flüssigmedium (1) auch einen Teil oder alle nicht-stärkehaltigen festen Bestandteile der Stärkequelle. Dies bedingt oftmals den Eintrag eines nicht zu vernachlässigenden Anteils an Phytat, z. B. aus der Kornfrucht. Um die daraus resultierende inhibierende Wirkung zu vermeiden, wird vorteilhafterweise in Schritt a2) dem Flüssigmedium mindestens eine Phytase zugesetzt, bevor das zuckerhaltige Flüssigmedium einem Fermentationsschritt zugeführt wird.

Die Zugabe der Phytase kann vor, während oder nach der Verflüssigung oder der Verzuckerung erfolgen, sofern sie die jeweils erforderliche Hitzestabilität aufweist.

Es können beliebige Phytasen eingesetzt werden, soweit deren Aktivität unter den Reaktionsbedingungen jeweils höchstens unwesentlich eingeschränkt ist. Bevorzugt sind Phytasen mit einer Temperaturstabilität (T50) > 50 °C und besonders bevorzugt > 60 °.C.

Die Menge an Phytase beträgt üblicherweise 1 bis 10000 Units/kg Stärkequelle und insbesondere 10 bis 4000 Units/kg Stärkequelle.

Zur Erhöhung der Gesamtzuckerausbeute bzw. zur Gewinnung freier Aminosäuren können dem Reaktionsgemisch während der Herstellung des zuckerhaltigen Flüssigmediums außerdem weitere Enzyme, zum Beispiel Pullulanasen, Cellulasen, Hemicellulasen, Glucanasen, Xylanasen, Glucosidasen oder Proteasen, zugesetzt werden. Der Zusatz dieser Enzyme kann die Viskosität positiv beeinflussen, d. h. herabsetzen (z. B. durch Spaltung langkettiger (auch bezeichnet als längerkettiger) Glucane und/oder von (Arabino-)Xylanen), die Freisetzung metabolisierbarer Glucoside und die Freisetzung von (Rest-)Stärke bewirken. Der Einsatz von Proteasen hat analoge positive Effekte, wobei zusätzlich Aminosäuren als Wachstumsfaktoren für die Fermentation freigesetzt werden können.

Durch die hier beschriebene Anwendung der Schritte a1) und a2) stellt man, je nachdem ob eine Verzuckerung durchgeführt wurde oder nicht, ein Dextrin- bzw. Monooder Disaccharid enthaltendes Flüssigmedium mit einem Gesamtkonzentration an Mono- Di-, und/oder Oligosacchariden in den oben genannten Bereichen her.

Bei den im Flüssigmedium (1) nach Verzuckerung enthaltenen Zuckern handelt es sich insbesondere um Glucose, wobei auch weitere Monosaccharide wie von Glucose verschiedene Hexosen und Pentosen, z. B. Fructose, Mannose, Galactose, Sorbose, Xylose, Arabinose und Ribose enthalten sein können. Der Anteil an von Glucose verschiedenen Monosacchariden kann abhängig von der verwendeten Stärkequelle und den darin enthaltenen nicht-stärkehaltigen Bestandteilen variieren und durch die Verfahrensführung, beispielsweise durch Aufschluss von Cellulosebestandteilen durch Zusatz von Cellulasen; beeinflusst werden. Vorteilhafterweise umfassen die Monosaccharide des zuckerhaltigen Flüssigmediums einen Anteil an Glucose von mindestens 60 Gew.-%, häufig mindestens 70 Gew.-%, insbesondere mindestens 80 Gew.-% und speziell mindestens 85 Gew.-%, bezogen auf die in dem zuckerhaltigen Flüssigmedium enthaltene gesamte Zuckermenge. Üblicherweise liegt der Glucoseanteil im Bereich von 75 bis 99,9 Gew.-%, insbesondere von 80 bis 99 Gew.-% und speziell von 85 bis 97 Gew.-%, bezogen auf die in dem zuckerhaltigen Flüssigmedium enthaltene gesamte Zuckermenge. Sofern keine Verzuckerung durchgeführt wurde, entspricht der Anteil an Dextrinen an den im Medium (1) enthaltenen Mono-, Di- und Oligosacchariden im Wesentlichen dem Glucoseanteil.

Sofern keine Verzuckerung durchgeführt wurde, liegen die verstoffwechselbaren Glucoseäquivalente im Wesentlichen in Form von Oligosacchariden, insbesondere Dextrinen vor. Hauptbestandteil dieser Oligosacchariden bzw. Dextrine ist typischerweise Glucose, wobei das Medium auch geringe Mengen an Mono- und/oder Disacchariden und aus anderen Monosaccharid-Einheiten aufgebaute Oligosaccharid-Einheiten enthalten kann. Typischerweise umfassen die zuckerhaltigen Bestandteile im Flüssigmediums (1), d. h. die Mono- Di- und Oligosaccharide, dann einen Anteil an Oligosacchariden, insbesondere Dextrinen, von mindestens 60 Gew.-%, häufig mindestens 70 Gew.-% insbesondere mindestens 80 Gew.-%, speziell mindestens 90 Gew.-%, d. h. der Anteil der Mono- und Disaccharide ist weniger als 40 Gew.-%, häufig weniger als 30 Gew.-%, insbesondere weniger als 20 Gew.-% und speziell weniger als 10 Gew.-%. Üblicherweise liegt der Anteil an Glucose, in freier oder gebundener Form, unter den Glukoseäquivalenten des Mediums (1) im Bereich von 50 bis 99 Gew.-%, insbesondere von 75 bis 97 Gew.-% und speziell von 80 bis 95 Gew.-%, bezogen auf die gesamte Menge an Glukoseäquivalenten.

Erfindungsgemäß erfolgt die anschließende Fermentation sowohl unter Verwendung des Flüssigmediums (1) als auch einer davon verschiedenen Quelle an verstoffwechselbaren Mono-, Di- und/oder Oligosacchariden (im Folgenden auch Zuckerquelle). Die hierzu verwendeten Mono-, Di- und/oder Oligosaccharide können sowohl als solche oder in Form einer Zusammensetzung eingesetzt werden, die verstoffwechselbare Mono-, Di- und/oder Oligosaccharide in einer Konzentration von wenigstens 50 Gew.-% vorzugsweise in einer Konzentration von wenigstens 60 Gew.-%, bezogen auf das Gesamtgewicht des Mediums, enthält und die im Unterschied zu dem wässrigen Flüssigmedium (1) im Wesentlichen frei ist von in Wasser unlöslichen Feststoffen.

Die in der Zuckerquelle enthaltenen Mono-, Di- oder Oligosacchariden sind vorzugsweise unter Monosacchariden, in der Regel Hexosen und/oder Pentosen, z. B. Glucose, Fructose, Mannose, Galactose, Sorbose, Xylose, Arabinose und Ribose, speziell unter Glucose, Fructose und Galactose, und Disacchariden wie Saccharose, Maltose, Lactose, speziell Saccharose, ausgewählt. Geeignet sind auch Mischungen von Monosacchariden und Disacchariden, sowie Oligosaccharide mit einem hohen Anteil eingebauter Glucose und deren Mischungen mit Monosacchariden und/oder Disacchariden. Beispiele für Zusammensetzungen, die verstoffwechselbare Mono-, Di- und/oder Oligosaccharide in einer Konzentration von wenigstens 50 Gew.-% enthalten und die im Wesentlichen frei sind von in Wasser unlöslichen Feststoffen umfassen Glucosesirupe, Saccharosesirupe, Dicksäfte, Maltosesirupe, Dextrinsirupe aber auch Abfallprodukte der Zuckerherstellung (Melassen), insbesondere Melassen aus der Rübenzuckerherstellung sowie Melassen aus der Rohrzuckerherstellung.

Bevorzugt sind insbesondere Substanzen, die überwiegend Mono- und/oder Disaccharide, insbesondere Glucose und/oder Saccharose enthalten, sowie Mischungen, die Glucose und/oder Saccharose und Oligosaccharide mit einem hohen Anteil eingebauter Glucose enthalten, z. B. Glucose, Saccharose, Glucosesirupe, Saccharosesirupe, Dicksäfte und Melassen.

Die Mono-, Di- und/oder Oligosaccharide beziehungsweise die sie enthaltenden Zusammensetzungen können ebenso wie das Flüssigmedium (1) sowohl zum Ansetzen der Fermentationsmediums (Batchphase) als auch zum Zufüttern während der Fermentation, wenn diese als Fed-Batch, bzw. semikontinuierlich durchgeführt wird, eingesetzt werden.

Die durch Zugabe des Flüssigmediums (1) in die Fermentation eingebrachte Gesamtmenge an Mono-, Di- und/oder Oligosacchariden, macht vorzugsweise wenigstens 40 Gew.-%, insbesondere wenigstens 50 Gew.-%, besonders bevorzugt wenigstens 60 Gew.-%, z. B. 40 bis 95 Gew.-%, insbesondere 50 bis 90 Gew.-% und speziell 60 bis 90 Gew.-% der Gesamtmenge der in die Fermentation eingebrachten Mono-, Diund Oligosacchariden aus.

Flüssigmedium (1) und die Mono-, Di- und/oder Oligosaccharide, bzw. die sie enthaltenen Zusammensetzungen können separat voneinander oder zusammen der Fermentation zugeführt werden.

Gemäß einer bevorzugten Ausführungsform der Erfindung werden Flüssigmedium (1) und die Mono-, Di- und/oder Oligosaccharide bzw. die sie enthaltende Zusammensetzung vor der Zugabe zur Fermentation miteinander vermischt. Auf diese Weise wird bei Einsatz eines niedrig konzentrierten Flüssigmediums, das in der Regel eine Gesamtkonzentration an Mono-, Di- und Oligosacchariden von 100 bis 400 g/kg aufweist, der Gesamtzuckergehalt des nach den Schritten a1) und a2) erhaltenen zuckerhaltigen Flüssigmediums (1) erhöht, und zwar um wenigstens 50 g/kg, insbesondere wenigstens 100 g/kg, speziell wenigstens 150 g/kg, z. B. um 50 bis 300 g/kg, insbesondere um 100 bis 250 g/kg und speziell um 120 bis 200 g/kg auf mehr als 40 Gew.-%, bevorzugt auf wenigstens 45 Gew.-%, insbesondere auf wenigsten 50 Gew.-% und besonders bevorzugt auf wenigstens 55 Gew.-%, bezogen auf das Gesamtgewicht.

Nach der Zugabe dieser Zuckerquellen zum Flüssigmedium (1) weist das resultierende Flüssigmedium vorzugsweise einen Trockenmassegehalt im Bereich von 45 bis 80 Gew.-% und besonders bevorzugt im Bereich von 50 bis 75 Gew.-% oder 55 bis 75 Gew.-%, bezogen auf das Gesamtgewicht, auf. Hierbei ist es vorteilhaft, die Viskosität des Flüssigmediums (1), z. B. mittels Temperatureinstellung, so zu regulieren, dass Werte von maximal 20 Pas, besonders bevorzugt maximal 15 Pas und ganz besonders bevorzugt maximal 8 Pas nicht überschritten werden.

In einer bevorzugten Ausführungsform der Erfindung werden die Mono- und/oder Disaccharide in Form eines Glucose oder Saccharose enthaltenden Nebenprodukts der Zuckerherstellung zu dem ersten Flüssigmedium (1) gegeben. Beispiele hierfür sind die bei der Zuckerherstellung aus Rohr- oder insbesondere Rübenzucker anfallende Melassen.

Erfindungsgemäß wird das in den Schritten a1) und a2) hergestellte Flüssigmedium (1) und die davon verschiedenen Zuckerquellen einer Fermentation zugeführt, wo sie zur Kultivierung der Mikroorganismen dienen. In der Fermentation werden die mikrobiellen Stoffwechselprodukte von den Mikroorganismen produziert.

Die Durchführung der Fermentation kann in der dem Fachmann bekannten üblichen Art und Weise erfolgen. Hierzu wird in man der Regel den jeweils gewünschten Mikroorganismus in dem nach dem hier beschriebenen Verfahren erhaltenen Flüssigmedium kultivieren.

Das Fermentationsverfahren kann sowohl diskontinuierlich (Batchfahrweise) als auch semikontinuierlich (Fed-Batch-Fahrweise, einschließlich Fedbatch mit Zwischenernten) betrieben werden, wobei die semikontinuierliche Fahrweise bevorzugt ist.

Beispielsweise kann man das nach dem erfindungsgemäßen Verfahren erhaltene Flüssigmedium (1) oder eine konventionelle Zuckerquelle, d. h. verstoffwechselbare Mono-, Di- und/oder Oligosaccharide oder die Zusammensetzung, welche verstoffwechselbare Mono-, Di- und/oder Oligosaccharide in einer Konzentration von wenigstens 50 Gew.-% enthält und welche typischerweise im Wesentlichen frei ist von in Wasser unlöslichen Feststoffen oder deren Mischung, gegebenenfalls nach Verdünnen mit Wasser und Zugabe üblicher Medienbestandteile wie Puffer, Nährsalze, Stickstoffquellen wie Ammoniumsulfat, Harnstoff etc., komplexe Nährmedienbestandteile, enthaltend Aminosäuren, wie Hefeextrakte, Peptone, CSL und dergleichen, mit dem gewünschten Mikroorganismus inokulieren, und diesen unter Fermentationsbedingungen vermehren, bis die Mikroorganismenkonzentration den für die Fermentation gewünschten stationären Zustand erreicht. Hierbei wird der in dem Flüssigmedium (1) enthaltene Zucker verstoffwechselt und das gewünschte Stoffwechselprodukt gebildet (so genannte Batchfahrweise oder Batchphase).

Bei der Fed-Batch-Fahrweise wird dann durch Zufuhr von weiterem, nach dem erfindungsgemäßen Verfahren erhältlichem Flüssigmedium (1) und der davon verschiedenen Zuckerquelle, insbesondere durch Zufuhr des durch Vermischen des Flüssigmediums (1) mit der davon verschiedenen Zuckerquelle erhaltenen Flüssigmediums der Fermentationsprozess weiter fortgeführt und das vom Mikroorganismus überproduzierte Stoffwechselprodukt reichert sich in der Fermentationsbrühe an, wobei das Stoffwechselprodukt sowohl in den Zellen des Mikroorganismus als auch in der wässrigen Phase des Fermentationsmediums vorliegen kann.

Vorzugsweise wird man die Fermentation semikontinuierlich, d. h. als Fed-Batch durchführen. Dabei wird man so vorgehen, dass man den Mikroorganismus zunächst unter Verwendung eines zuckerhaltigen Flüssigmediums, beispielsweise unter Verwendung eines Flüssigmediums (1) oder einer anderen Zuckerquelle, vermehrt, bis die gewünschte Mikroorganismenkonzentration im Fermenter erreicht ist. Anschließend werden das Flüssigmedium (1) zusammen mit der weiteren Zuckerquelle, d. h. verstoffwechselbaren Mono-, Di- und/oder Oligosacchariden oder einem Medium, das verstoffwechselbare Mono-, Di- und/oder Oligosaccharide in einer Konzentration von wenigstens 50 Gew.-% enthält und das im Wesentlichen frei ist von in Wasser unlöslichen Feststoffen, dem Fermenter zugeführt. Hierdurch wird der Fermentationsprozess aufrechterhalten und das vom Mikroorganismus überproduzierte Stoffwechselprodukt reichert sich in der Fermentationsbrühe an (s. o.). Dabei liegt das Volumenverhältnis von zugeführtem zuckerhaltigen Flüssigmedium (1) und der weiteren Zuckerquelle, zu dem vorgelegten und die Mikroorganismen enthaltenden Batch-Medium im Allgemeinen im Bereich von etwa 1:10 bis 10:1 und vorzugsweise bei etwa 1:5 bis 5:1 und speziell im Bereich von 1:1 bis 5:1. Insbesondere über die Zufuhrgeschwindigkeit des zuckerhaltigen Flüssigmediums kann der Zuckergehalt in der Fermentationsbrühe reguliert werden. In der Regel wird man die Zufuhrgeschwindigkeit so einstellen, dass der Monosaccharidgehalt in der Fermentationsbrühe im Bereich von > 0 Gew.-% bis etwa 5 Gew.-% liegt und insbesondere einen Wert von 3 Gew.-% nicht überschreitet.

Das in Schritt a2) erhaltene, zuckerhaltige Flüssigmedium oder dessen Mischung mit der weiteren Zuckerquelle kann vor der Fermentation gegebenenfalls sterilisiert werden, wobei man die Mikroorganismen üblicherweise durch thermische oder chemische Verfahren abtötet. Hierzu heizt man das zuckerhaltige Flüssigmedium üblicherweise auf Temperaturen oberhalb 80 °C auf. Die Abtötung bzw. Lyse der Zellen kann unmittelbar vor der Fermentation erfolgen. Hierzu wird die gesamte zuckerhaltige Flüssigmedium der Lyse bzw. Abtötung zugeführt. Diese kann thermisch, mechanisch oder chemisch erfolgen.

Es versteht sich für den Fachmann, dass das auf fermentativem Weg hergestellte Lysin jeweils in der von den eingesetzten Mikroorganismen produzierten Enantiomerenform erhalten wird. So erhält man in der Regel das L-Enantiomer des Lysins.

Die in der Fermentation eingesetzten Mikroorganismen können natürlichen Ursprungs oder genetisch modifiziert sein. Beispiele für geeignete Mikroorganismen und Fermentationsverfahren sind z. B. *Corynebacterium glutamicum* und die von Ikeda geschriebenen Verfahren (siehe: Ikeda, M.: Amino Acid Production Process (2003) Adv. Blochem. Engin/Biotechnol 79, 1-35).

Geeignete Mikroorganismen sind üblicherweise ausgewählt unter den Gattungen Corynebacterium, Bacillus, Ashbya, Escherichia, Aspergillus, Alcaligenes, Actinobacillus, Anaerobiospirillum, Lactobacillus, Propionibacterium, Rhizopus und Clostridium, insbesondere unter Stämmen von Corynebacterium glutamicum, Bacillus subtilis, Ashbya gossypii, Escherichia coli, Aspergillus niger oder Alcaligenes latus, Anaerobiospirillum succiniproducens, Actinobacillus succinogenes, Lactobacillus delbrückii, Lactobacillus leichmannii, Propionibacterium arabinosum, Propionibacterium schermanii, Propionibacterium freudenreichii, Clostridium propionicum, Clostridium formicoaceticum, Clostridium acetobutylicum, Rhizopus arrhizus und Rhizopus oryzae.

In einer bevorzugten Ausführungsform handelt es sich bei dem in der Fermentation eingesetzten Mikroorganismus um einen Stamm aus der Gattung Corynebacterium, insbesondere um einen Stamm des Corynebacterium glutamicum. Insbesondere handelt es sich um einen Stamm der Gattung Corynebacterium, speziell des Corynebacterium glutamicum, welcher Lysin überproduziert.

In einer weiteren bevorzugten Ausführungsform handelt es sich bei dem in der Fermentation eingesetzten Mikroorganismus um einen Stamm aus der Gattung Escherichia, insbesondere um einen Stamm des Escherichia coli. Insbesondere handelt es sich um einen Stamm der Gattung Escherichia, speziell des Escherichia coli, welcher Lysin überproduziert.

Gemäß der Erfindung handelt es sich bei dem in der Fermentation von den Mikroorganismen produzierten Stoffwechselprodukt um Lysin. Zur Durchführung der Fermentation können hier analoge Bedingungen und Vorgehensweisen angewendet werden, wie sie für andere Kohlenstoffquellen beschrieben wurden, z. B. in Pfefferle et al., a.a.O., und US 3,708,395. Prinzipiell kommen sowohl eine kontinuierliche als auch eine diskontinuierliche (Batch oder Fed-Batch) Betriebsweise in Betracht, bevorzugt ist die Fed-Batch Betriebsweise.

Bei der Fermentation resultiert eine Fermentationsbrühe, die neben dem gewünschten mikrobiellen Stoffwechselprodukt, d. R. Lysin, im Wesentlichen die während der Fermentation erzeugte Biomasse, die nicht verstoffwechselten Bestandteile der verzuckerten Stärkelösung und insbesondere die nicht-stärkehaltigen festen Bestandteile der Stärkequelle, wie z. B. Fasern und nicht verwertete Zucker, sowie nicht verwertete Puffer- und Nährsalze enthält. Dieses Flüssigmedium wird in der vorliegenden Anmeldung auch als Fermentationsbrühe bezeichnet, wobei der Ausdruck Fermentationsbrühe auch das (zuckerhaltige) Flüssigmedium umfasst, bei dem eine erst teilweise oder unvollständige fermentative Umsetzung der darin enthaltenen Zucker, d. h. eine teilweise oder unvollständige mikrobielle Verstoffwechselung der verwertbaren Zucker (z. B. Mono- und Disaccharide), erfolgt ist.

Vor der Isolierung oder Abreicherung des mikrobiellen Stoffwechselprodukts, d. R. des Lysins, oder Abtrennung der flüchtigen Bestandteile der Fermentationsbrühe, wird gegebenenfalls ein Sterilisierungsschritt in der oben beschriebenen Weise durchgeführt.

Eine spezielle Ausführungsform der Erfindung betrifft ein Verfahren, bei dem man das mikrobielle Stoffwechselprodukt, d. R. Lysin, aus der Fermentationsbrühe abreichert oder isoliert. Die flüchtigen Bestandteile der Fermentationsbrühe werden anschließend weitgehend entfernt, wobei man eine feste oder halbfeste Proteinzusammensetzung erhält. Eine genauere Beschreibung zur Durchführung eines solchen Verfahrens und der dabei erhaltenen Proteinzusammensetzung ist Gegenstand der WO 2005/116228 (PCT/EP2005/005728) der Anmelderin.

Die Isolierung oder Abreicherung des Stoffwechselprodukts, d. R. des Lysins, aus der Fermentationsbrü- he erfolgt in der Regel derart, dass man wenigstens ein Stoffwechselprodukt so aus der Fermentationsbrühe abreichert oder isoliert, dass der Gehalt dieses Stoffwechselprodukts in der verbleibenden Fermentationsbrühe höchstens 20 Gew.-%, insbesondere höchstens 10 Gew.-%, speziell höchstens 5 Gew.-% und ganz speziell höchstens 2,5 Gew.-%, jeweils bezogen auf das Gesamtgewicht der verbleibenden Fermentationsbrühe, beträgt.

Die Isolierung oder Abreicherung des mikrobiellen Stoffwechsetprodukts, d. R. des Lysins, aus der Fermentationsbrühe kann in einem oder mehreren Schritten erfolgen. Ein wesentlicher Schritt dabei ist die Abtrennung der festen Bestandteile aus der Fermentationsbrühe. Diese kann entweder vor oder nach der Isolierung des Wertprodukts durchgeführt werden. Sowohl zur Isolierung von Wertstoffen als auch zur Abtrennung von Feststoffen, d. h. Fest-Flüssig-Phasenseparation, sind im Fachgebiet übliche Methoden bekannt, die auch Schritte zur Grob- und Feinreinigung der Wertstoffe sowie zur Konfektionierung umfassen (z. B. beschrieben in Belter, P. A, Bioseparations: Downstream Processing for Biotechnology, John Wiley &.Sons (1988), und Ullmann's Encyclopedia of Industrial Chemistry, 5. Aufl. auf CD-ROM, Wiley-VCH).

Zur Isolierung des Wertprodukts, d. R. des Lysins, kann man vorteilhafterweise so vorgehen, dass man zunächst die festen Bestandteile aus der Fermentationsbrühe entfernt, z. B. mittels Zentrifugation oder Filtration, und anschließend das Wertprodukt aus der flüssigen Phase isoliert, z. B. durch Kristallisation, Fällung oder Adsorption. Alternativ kann das Wertprodukt auch direkt aus der Fermentationsbrühe isoliert werden, z. B. durch Einsatz chromatographischer Verfahren oder von Extraktionsverfahren. Als chromatographisches Verfahren ist insbesondere die Ionenaustauschchromatographie zu nennen, bei der das Wertprodukt selektiv auf der Chromatographiesäule isoliert werden kann. In diesem Fall erfolgt die Abtrennung der Feststoffe aus der verbleiben-den Fermentationsbrühe vorteilhafterweise z. B. durch Dekantieren, Eindampfen oder/und Trocknung.

Eine weitere spezielle Ausführungsform betrifft ein Verfahren, bei dem man die flüchtigen Bestandteile der Fermentationsbrühe ohne vorherige Isolierung oder Abreicherung des Lysins, und gegebenenfalls ohne vorherige Abtrennung fester Bestandteile, weitgehend oder vollständig entfernt, wobei man eine feste Formulierung des Lysins erhält. Eine genauere Beschreibung zur Durchführung eines solchen Verfahrens findet sich in der PCT/EP2006/066057 (ältere Patentanmeldung DE 102005042541.0) der Anmelderin.

Weitgehend bedeutet, dass nach Entfernung der flüchtigen Bestandteile ein fester oder zumindest halbfester Rückstand verbleibt, der sich gegebenenfalls durch Zusatz von Feststoffen in ein festes Produkt überführen lässt. In der Regel bedeutet dies, die flüchtigen Bestandteile bis zu einem Restfeuchtegehalt von nicht mehr als 30 Gew.-%, häufig nicht mehr als 20 Gew.-% und insbesondere nicht mehr als 15 Gew.-%, zu entfernen. In der Regel wird man die flüchtigen Bestandteile der Fermentationsbrühe vorteilhafterweise bis auf einen Restfeuchtigkeitsgehalt im Bereich von 0,2 bis 30 Gew.-%, bevorzugt 1 bis 20 Gew.-%, besonders bevorzugt 2 bis 15 Gew.-% und ganz besonders bevorzugt 5 bis 15 Gew.-%, bezogen auf das nach Trocknung ermittelte Gesamtgewicht der festen Bestandteile, aus der Fermentationsbrühe entfernen. Der Restfeuchtigkeitsgehalt kann durch übliche dem Fachmann bekannte Verfahren bestimmt werden, z. B. mittels Thermogravimetrie (Hemminger et al., Methoden der thermischen Analyse, Springer Verlag, Berlin, Heidelberg, 1989).

Die Gewinnung des Lysins in fester Form aus der Fermentationsbrühe kann in ein, zwei oder mehreren Stufen erfolgen, insbesondere in einer ein- oder zweistufigen Vorgehensweise. In der Regel wird mindestens eine, insbesondere die abschließende Stufe zur Gewinnung des Lysins in fester Form einen Trocknungsschritt umfassen.

Bei der einstufigen Vorgehensweise wird man die flüchtigen Bestandteile der Fermentationsbrühe, gegebenenfalls nach vorgenannter Vorabtrennung, entfernen, bis der gewünschte Restfeuchtigkeitsgehalt erreicht ist.

Bei der zwei- oder mehrstufigen Vorgehensweise wird man die Fermentationsbrühe, zunächst aufkonzentrieren, z. B. mittels (Mikro-, Ultra-)Filtration oder thermisch durch Verdampfen eines Teils der flüchtigen Bestandteile. Der Anteil der flüchtigen Bestandteile, die in dieser Stufe entfernt werden beträgt in der Regel 10 bis 80 Gew.-% und insbesondere 20 bis 70 Gew.-%, bezogen auf das Gesamtgewicht der flüchtigen Bestandteile der Fermentationsbrühe. In einer oder mehreren sich anschließenden Stufen werden die restlichen flüchtigen Bestandteile der Fermentationsbrühe entfernt, bis der gewünschte Restfeuchtigkeitsgehalt erreicht ist.

Das Entfernen der flüchtigen Bestandteile des Flüssigmediums erfolgt gemäß dieser Ausführungsform im Wesentlichen ohne eine vorherige Abreicherung oder gar Isolierung des Wertprodukts. Folglich wird bei der Entfernung der flüchtigen Bestandteile der Fermentationsbrühe das Lysin im Wesentlichen nicht zusammen mit den flüchtigen Bestandteilen des Flüssigmediums entfernt, sondern verbleibt mit wenigstens einem Teil, üblicherweise mit der Hauptmenge und insbesondere mit der Gesamtmenge der sonstigen festen Bestandteile aus der Fermentationsbrühe im so erhaltenen Rückstand. Dementsprechend können aber auch - vorzugsweise geringe - Anteile des Lysins, in der Regel maximal 20 Gew.-%, z. B. 0,1 bis 20 Gew.-%, bevorzugt nicht mehr als 10, insbesondere nicht mehr als 5 Gew.-%, besonders bevorzugt maximal 2,5 Gew.-% und ganz besonders bevorzugt maximal 1 Gew.-%, bezogen auf das Gesamttrockengewicht des Lysins, beim Entfernen der flüchtigen Bestandteile der Fermentationsbrühe zusammen mit diesen entfernt werden. In einer ganz besonders bevorzugten Ausführungsform verbleibt das Lysin zu wenigstens 90 Gew.-%, insbesondere wenigstens 95 Gew.-%, speziell 99 Gew.-% und ganz speziell etwa 100 Gew.-%, jeweils bezogen auf das Gesamttrockengewicht des Lysins, als Feststoff in Mischung mit dem nach Entfernen der flüchtigen Bestandteile erhaltenen Teil oder der Gesamtheit der festen Bestandteile des Fermentationsmediums.

Sofern gewünscht, kann vor dem Entfernen der flüchtigen Bestandteile ein Teil, z. B. 5 bis 80 Gew.-% und insbesondere 30 bis 70 Gew.-%, der nicht-stärkehaltigen festen Bestandteile aus der Fermentationsbrühe abgetrennt werden, z. B. mittels Zentrifugation oder Filtration. Gegebenenfalls wird man eine solche Vorabtrennung durchführen, um gröbere Feststoffpartikel, die keine oder nur geringe Anteile an Lysin enthalten, zu entfernen. Zur Vorfiltration können dem Fachmann bekannte, übliche Verfahren, z. B. unter Verwendung grobmaschiger Siebe, Netze, Lochbleche, oder dergleichen angewendet werden. Gegebenenfalls kann eine Abtrennung grober Feststoffpartikel auch in einem Fliehkraftabscheider erfolgen. Die hierbei eingesetzten Apparaturen wie Dekanter, Zentrifugen, Sedikanter und Separatoren sind dem Fachmann ebenfalls bekannt. Auf diese Weise erhält man einen festen oder halbfesten, z. B. pastösen Rückstand, welcher das Lysin und die nichtflüchtigen, in der Regel festen nicht-stärkehaltigen Bestandteile der Stärkequelle oder zumindest große Teile davon, häufig wenigstens 90 Gew.-% oder die Gesamtmenge der festen nicht-stärkehaltigen Bestandteile enthält.

Durch Zugabe von Formulierungshilfsmitteln wie Träger- und Coatingmaterialien, Bindemitteln sowie anderer Additive können die Eigenschaften des getrockneten und zusammen mit den festen Bestandteilen der Fermentation vorliegenden Lysins gezielt hinsichtlich verschiedener Parameter wie Wirkstoffgehalt, Korngröße, Partikelform, Neigung zum Stauben, Hygroskopizität, Stabilität, insbesondere Lagerstabilität, Farbe, Geruch, Fließverhalten, Agglomerationsneigung, elektrostatischer Aufladung, Licht- und Temperaturempfindlichkeit, mechanischer Stabilität und Redispergierbarkeit in an sich bekannter Weise konfektioniert werden.

Zu den üblicherweise verwendeten Formulierungshilfsmitteln gehören z. B. Bindemittel, Trägermaterialien, Puderungs-/Fließhilfsmittel, ferner Farbpigmente, Biozide, Dispergiermittel, Antischaummittel, viskositätsregulierende Mittel, Säuren, Laugen, Antioxidantien, Enzymstabilisatoren, Enzyminhibitoren, Adsorbate, Fette, Fettsäuren, Öle oder Mischungen hieraus. Derartige Formulierungshilfsmittel werden vorteilhaft insbesondere bei Anwendung von Formulierungs- und Trocknungsverfahren wie Sprüh-, Wirbelschicht- und Gefriertrocknung als Trocknungshilfsmittel eingesetzt. Wegen weiterer Details wird auf die PCT/EP2006/066057 (ältere Anmeldung DE 102005042541.0) verwiesen

Der Anteil an den vorgenannten Zusatzstoffen und gegebenenfalls weiteren Zusatzstoffen wie Coatingmaterialien kann nach den Erfordernissen des Lysins sowie in Abhängigkeit von den Eigenschaften der eingesetzten Zusatzstoffe stark variieren und z. B. im Bereich von 0,1 bis 80 Gew.-% und insbesondere im Bereich von 1 bis 30 Gew.-%, jeweils bezogen auf das Gesamtgewicht des fertig formulierten Produkts bzw. Stoffgemischs, liegen.

Die Zugabe von Formulierungshilfsmifteln kann vor, während oder nach der Aufarbeitung der Fermentationsbrühe (auch als Produktformulierung oder Feststoffdesign bezeichnet) und insbesondere während der Trocknung erfolgen. Eine Zugabe von Formulierungshilfsmitteln vor Aufarbeitung der Fermentationsbrühe bzw. des Lysins kann insbesondere vorteilhaft sein, um die Prozessierbarkeit der aufzuarbeitenden Stoffe bzw. Produkte zu verbessern. Die Formulierungshilfsmittel können sowohl dem in fester Form erhaltenen Lysin als auch einer Lysin enthaltenden Lösung oder Suspension zugegeben werden, z. B. nach abgeschlossener Fermentation direkt zu der Fermentationsbrühe oder zu einer im Verlauf der Aufarbeitung erhaltenen Lösung oder Suspension vor dem abschließenden Trocknungsschritt.

So können die Hilfsstoffe z. B. in eine Suspension des Lysins eingemischt werden; eine solche Suspension kann auch auf ein Trägermaterial gegeben werden, z. B. durch Aufsprühen oder Untermischen. Die Zugabe von Formulierungshilfsstoffen während der Trocknung kann z. B. eine Rolle spielen, wenn eine das Lysin enthaltende Lösung oder Suspension versprüht wird. Insbesondere nach der Trocknung erfolgt eine Zugabe von Formulierungshilfsmitteln z. B. beim Aufbringen von Überzügen bzw. Coatings/Coatingschichten auf getrocknete Partikel. Sowohl nach dem Trocknen als auch nach einem eventuellen Coatingschritt können dem Produkt weitere Hilfsmittel zugegeben werden.

Das Entfernen der flüchtigen Bestandteile aus der Fermentationsbrühe erfolgt in an sich bekannter Weise durch übliche Verfahren zur Abtrennung fester Phasen von flüssigen Phasen, einschließlich Filtrationsverfahren und Verfahren zum Verdampfen flüchtiger Bestandteile der flüssigen Phasen. Derartige Verfahren, die auch Schritte zur Grobreinigung der Wertstoffe sowie Schritte zur Konfektionierung umfassen können, werden z. B. in Belter, P. A, Bioseparations: Downstream Processing for Biotechnology, John Wiley & Sons (1988), und Ullmann's Encyclopedia of Industrial Chemistry, 5. Aufl. auf CD-ROM, Wiley-VCH, beschrieben. Im Rahmen der Produktformulierung bzw. der Aufarbeitung nach abgeschlossener Fermentation anwendbare, dem Fachmann bekannte Verfahren, Apparaturen, Hilfsstoffe bzw. allgemeine und spezielle Ausführungsformen sind ferner in EP 1038 527, EP 0648 076, EP 835613, EP 0219 276, EP 0394 022, EP 0547 422, EP 1088 486, WO 98/55599, EP 0758 018 und WO 92/12645 beschrieben.

In einer ersten Variante dieser Ausführungsform wird man das Lysin, sofern es in der flüssigen Phase gelöst vorliegt, aus der flüssigen Phase in die feste Phase überführen, z. B. durch Kristallisation oder Fällung. Anschließend erfolgt eine Abtrennung der nichtflüchtigen festen Bestandteile einschließlich des Lysins, z. B. mittels Zentrifugation, Dekantieren oder Filtration.

In einer zweiten Variante dieser Ausführungsform werden die flüchtigen Bestandteile durch Verdampfen entfernt. Das Verdampfen kann in an sich bekannter Weise erfolgen. Beispiele für geeignete Verfahren zum Verdampfen flüchtiger Bestandteile sind die Sprühtrocknung, Wirbelschichttrocknung bzw. -agglomeration, Gefriertrocknung, Strom- und Kontakttrocknern sowie Extrusionstrocknung. Auch eine Kombination der vorgenannten Verfahren mit formgebende Verfahren wie Extrusion, Pelletierung oder Prilling kann vorgenommen werden. Bei diesen letztgenannten Verfahren werden vorzugsweise teilweise oder weitgehend vorgetrocknete Lysin-haltige Stoffgemische eingesetzt.

In einer bevorzugten Ausführungsform umfasst das Entfernen der flüchtigen Bestandteile der Fermentationsbrühe ein Verfahren zur Sprühtrocknung oder ein Verfahren der Wirbelschichttrocknung, einschließlich der Wirbelschichtgranulierung. Hierzu wird die Fermentationsbrühe, gegebenenfalls nach einer Vorabtrennung zur Entfernung grober Feststoffpartikel, die keine oder nur geringe Anteile an Lysin enthalten, einer oder mehreren Sprüh- oder Wirbelschichttrocknungsapparaturen zugeführt. Der Transport bzw. die Zuführung der feststoffbeladenen Fermentationsbrühe erfolgt zweckmäßigerweise mittels üblicher Transportvorrichtungen für feststoffhaltige Flüssigkeiten, z. B. Pumpen wie Exzenterschneckenpumpen (z. B. der Fa. Delasco PCM) oder Hochdruckpumpen (z. B. der Fa. LEWA Herbert Ott GmbH).

Die Durchführung einer Fermentation kann auch derart erfolgen, dass man
(i) dem in Schritt a2) erhaltenen Flüssigmedium (1) oder dessen Mischung mit der weiteren Zuckerquelle eine Teilmenge von nicht mehr als 50 Gew.-%, z. B. im Bereich von 5 bis 45 Gew.-%, bezogen auf das Gesamtgewicht, entnimmt und die Restmenge, gegebenenfalls zusammen mit einer weiteren Zuckerquelle, wie oben definiert, einer Fermentation zur Herstellung eines Lysins (A) zuführt; und
(ii) die entnommene Teilmenge, gegebenenfalls nach vorheriger vollständiger oder teilweiser Abtrennung der nicht-stärkehaltigen festen Bestandteile der Stärkequelle, gegebenenfalls zusammen mit einer weiteren Zuckerquelle, wie oben definiert, einer Fermentation zur Herstellung eines weiteren Lysins (B) zuführt.

Im Falle der Abtrennung der nicht-stärkehaltigen festen Bestandteile gemäß (ii) beträgt der Feststoffgehalt des verbleibenden Anteils des Flüssigmediums bevorzugt maximal 50 Gew.-%, insbesondere maximal 30 Gew.-%, besonders bevorzugt maximal 10 Gew.-% und ganz besonders bevorzugt maximal 5 Gew.-%. Insbesondere ist es in diesem Fall bevorzugt, die gesamten Feststoffe vor der Fermentation zur Herstellung des weiteren Lysins (B) abzutrennen.

Diese Vorgehensweise ist insbesondere dann vorteilhaft, wenn für verschiedene Anwendungen des Lysins unterschiedliche Anforderungen an die Reinheit zu stellen sind. Demnach wird das Lysin (A), das z. B. als Futtermitteladditiv verwendet werden kann, unter Einsatz der feststoffhaltigen Fermentationsbrühe und das Lysin (B), das z. B. als Nahrungsmitteladditiv verwendet werden kann, unter Einsatz der gemäß (ii) feststoffabgereicherten Fermentationsbrühe hergestellt. Durch die vollständige oder teilweise Abtrennung der nicht-stärkehaltigen festen Bestandteile kann der Reinigungsaufwand bei der Aufarbeitung des Lysins, dessen Anwendungsbereich eine höhere Reinheit erfordert, z. B. als Nahrungsmitteladditiv, reduziert werden.

In einer weiteren bevorzugten Ausführungsform dieser Vorgehensweise kann z. B. wie folgt vorgegangen werden. Man implementiert eine vorzugsweise großvolumige Fermentation zur Herstellung von Lysin A, z. B. entsprechend den in der WO 2005/116228 (PCT/EP2005/005728) oder PCT/EP2006/066057 (die ältere Patentanmeldung DE 102005042541.0) beschriebenen Verfahren. Gemäß (i) wird ein Teil des in Schritt a2) erhaltenen Flüssigmediums (1) oder dessen Mischung mit der weiteren Zuckerquelle entnommen. Der gemäß (i) entnommene Teil kann gemäß (ii) durch übliche Verfahren, z. B. Zentrifugation oder Filtration, vollständig oder teilweise von den Feststoffen befreit werden, je nach den Erfordernissen der Fermentation zur Herstellung von Lysin B. Das so gewonnene, gegebenenfalls vollständig oder teilweise von den Feststoffen befreite Flüssigmedium (1) wird gemäß (ii), gegebenenfalls zusammen mit einer weiteren Zuckerquelle, wie oben definiert, einer Fermentation zur Produktion des Lysins B zugeführt. Ein gemäß (ii) abgetrennter Feststoffstrom wird vorteilhafterweise zum Strom des zuckerhaltigen Flüssigmediums der großvolumigen Fermentation zurück gegeben.

Die abgetrennten Feststoffe können im Rahmen des parallel betriebenen, großvolumigen Fermentationsverfahrens zusammen mit dem Lysin (A) gewonnen werden.

Nach der Trocknung und/oder Formulierung bzw. Konfektionierung können der Produktformulierung bzw. Proteinzusammensetzung ganze oder gemahlene Getreidekörner, bevorzugt Mais, Weizen, Gerste, Hirse, Triticale und/oder Roggen zugegeben werden.

Die nachfolgenden Beispiele dienen der Veranschaulichung einzelner Aspekte der vorliegenden Erfindung, sie sind jedoch in keiner Weise als einschränkend zu verstehen.

### Beispiele

### I. Vermahlen der Stärkequelle

Die im Folgenden eingesetzten Mahlgüter wurden wie folgt hergestellt. Ganze Maiskörner wurden unter Verwendung einer Rotormühle vollständig vermahlen. Unter Einsatz unterschiedlicher Schlägerwerke, Mahlbahnen bzw. Siebeinbauten wurden dabei drei verschiedene Feinheiten erzielt. Eine Siebanalyse des Mahlgutes mittels Labor-Vibrationssieb (Vibrations-Analysenmaschine: Retsch Vibrotronic Typ VE1; Siebzeit 5 min; Amplitude: 1,5 mm) ergab die in Tabelle I aufgeführten Ergebnisse.

**Tabelle I**

| **Versuchsnummer** | **T 70/03** | **T 71/03** | **T 72/03** |
|---|---|---|---|
| < 2mm/% | 99,4 | 100 | 100 |
| < 0,8mm/% | 66 | 100 | 99 |
| < 0,63 mm / % | 58,6 | 98,5 | 91 |
| < 0,315 mm / % | 48,8 | 89 | 65 |
| < 0,1 mm / % | 1 | 25 | 9,6 |
| < 0,04 mm / % | 1 | 8 | 3,2 |
| Mahlgutmenge gesamt | 20 kg | 11,45 kg | 13,75 kg |

### II. Enzymatische Stärkeverflüssigung und -verzuckerung

### II.1. Ohne Phytase im Verzuckerungsschritt

### II.1a) Enzymatische Stärkeverflüssigung

Aus 320 g trocken vermahlenem Maismehl (T71/03) wurde unter stetem Rühren eine Suspension in 480 g Wasser angesetzt und mit 310 mg Calciumchlorid versetzt. Das Rühren wurde während der gesamten Versuchsdurchführung fortgesetzt. Nach Einstellung des pH-Wertes mit H₂SO₄ auf 6,5 und Erhitzen auf 35 °C wurden 2,4 g Termamyl 120L Typ L (Novozymes A/S) zugegeben. Über 40 min wurde das Reaktionsgemisch auf eine Temperatur von 86,5 °C erhitzt, wobei der pH gegebenenfalls mit NaOH auf den zuvor eingestellten Wert nachjustiert wurde. Innerhalb von 30 min wurden weitere 400 g des trocken vermahlenen Maismehls (T71/03) zugegeben, wobei die Temperatur auf 91 °C erhöht wurde. Das Reaktionsgemisch wurde ca. 100 min bei dieser Temperatur gehalten. Anschließend wurden weitere 2,4 g Termamyl 120L zugegeben und die Temperatur ca. 100 min gehalten. Der Fortschritt der Verflüssigung wurde während der Durchführungsdauer mit der lod-Stärke-Reaktion verfolgt. Die Temperatur wurde abschließend auf 100 °C erhöht und das Reaktionsgemisch weitere 20 min gekocht. Zu diesem Zeitpunkt war keine Stärke mehr nachzuweisen. Der Reaktor wurde auf 35 °C abgekühlt.

### II.1b) Verzuckerung

Die aus II.1a) erhaltene Reaktionsmischung wurde unter stetem Rühren auf 61 °C erhitzt. Das Rühren wurde während der gesamten Versuchsdurchführung fortgesetzt. Nach Einstellung des pH-Wertes mit H₂SO₄ auf 4,3 wurden 10,8 g (9,15 ml) Dextrozyme GA (Novozymes A/S) zugegeben. Die Temperatur wurde ca. 3 h gehalten; wobei der Fortschritt der Reaktion mit Glucoseteststäbchen (S-Glucotest von Boehringer) verfolgt wurde. Die Ergebnisse sind in der unten stehenden Tabelle II aufgeführt. Anschließend wurde das Reaktionsgemisch auf 80 °C erhitzt und danach abgekühlt. Es wurden ca. 1180 g flüssiges Produkt mit einer Dichte von ca. 1,2 kg/l und einem mittels Infrarottrockner bestimmten Gehalt an Trockenmasse von etwa 53,7 Gew.-% erhalten. Der Gehalt an Trockenmasse (ohne wasserlösliche Inhaltsstoffe) betrug nach Waschen mit Wasser etwa 14 Gew.-%. Der durch HPLC bestimmte Anteil an Glucose im Reaktionsgemisch betrug 380 g/l (vgl. Tab. 2, Probe Nr. 7).

**Tabelle II**

| **Probe Nr.** | **min (ab Zugabe Glucoamylase)** | **Konz. Glucose im Uberstand [g/l]** |
|---|---|---|
| 1 | 5 ***** | 135 |
| 2 | 45 | 303 |
| 3 | 115 | 331 |
| 4 | 135 | 334 |
| 5 | 165 | 340 |
| 6 | 195 | 359 |
| 7 | 225 | 380 |

### II.2. Mit Phytase im Verzuckerungsschritt

### II.2a) Stärkeverflüssigung

Eine trocken vermahlene Maismehlprobe wurde entsprechend II.1a) verflüssigt.

### II.2b) Verzuckerung

Die aus II.2a) erhaltene Reaktionsmischung wurde unter stetem Rühren auf 61 °C erhitzt. Das Rühren wurde während der gesamten Versuchsdurchführung fortgesetzt. Nach Einstellung des pH-Wertes mit H₂SO₄ auf 4,3 wurden 10,8 g (9,15 ml) Dextrozyme GA (Novozymes A/S) und 70 µl Phytase (700 Units Phytase, Natuphyt Liquid 10000L von der BASF Aktiengesellschaft) zugegeben. Die Temperatur wurde ca. 3 h gehalten, wobei der Fortschritt der Reaktion mit Glucoseteststäbchen (S-Glucotest von Boehringer) verfolgt wurde. Anschließend wurde das Reaktionsgemisch auf 80 °C erhitzt und danach abgekühlt. Das erhaltene Produkt wurde mittels Infrarottrockner getrocknet und mit Wasser gewaschen. Der Anteil an Glucose im Reaktionsgemisch wurde durch HPLC bestimmt.

### II.3 Weitere Arbeitsvorschriften zur enzymatischen Stärkeverflüssigung und -verzuckerung

### II.3a) Maismehl

360 g deionisiertes Wasser wurden in einem Reaktionsgefäß vorgelegt. 1,54 ml CaCl₂ Stammlösung (100 g CaCl₂ x 2 H₂O / l) wurden bis zu einer Endkonzentration von etwa 70 ppm Ca²⁺ in der Maische zugegeben. Unter stetem Rühren ließ man 240 g Maismehl langsam in das Wasser einrieseln. Nach Einstellung des pH auf 6,5 mit 50 gew.-%iger wässriger NaOH-Lösung gab man 4,0 ml (= 2 Gew.-% Enzym/Trockenmasse) Termamyl 120 L Typ L (Novozymes A/S) zu. Die Maische wurde dann schnell auf bis zu 85 °C erhitzt. Hierbei musste der pH fortwährend kontrolliert und gegebenenfalls angepasst werden.

Nach Erreichen der endgültigen Temperatur begann man mit der Zugabe von weiterem Mehl, zunächst von 50 g Mehl. Zusätzlich gab man der Maische 0,13 ml CaCl₂ Stammlösung zu, um die Ca²⁺-Konzentration bei 70 ppm zu halten. Während der Zugabe hielt man die Temperatur konstant bei 85 °C. Man wartete mindestens 10 min ab, um eine vollständige Reaktion zu gewährleisten, bevor man eine weitere Portion (50 g Mehl und 0,13 ml CaCl₂ Stammlösung) zugab. Nach Zugabe von zwei Portionen gab man 1,67 ml Termamyl zu; anschließend gab man zwei weitere Portionen (jeweils 50 g Mehl und 0,13 ml CaCl₂ Stammlösung) zu. Es wurde ein Gehalt an Trockenmasse von 55 Gew.-% erreicht. Nach der Zugabe erhöhte man die Temperatur auf 100 °C und kochte die Maische 10 min.

Man entnahm eine Probe und kühlte diese auf Raumtemperatur. Nach Verdünnung der Probe mit deionisiertem Wasser (etwa 1:10) gab man einen Tropfen konzentrierte Lugolsche Lösung (Mischung von 5 g Iod und 10 g Kaliumiodid pro Liter) zu. Eine tiefblaue Färbung zeigte einen Gehalt an verbleibender Stärke an; eine Braunfärbung trat ein, wenn die Stärke vollständig hydrolysiert worden war. Wenn der Test einen Anteil verbleibender Stärke anzeigte, wurde die Temperatur erneut auf 85 °C erniedrigt und konstant gehalten. Man gab weitere 1,67 ml Termamyl zu, bis die lod-Stärke-Reaktion negativ ausfiel.

Die negativ auf Stärke getestete Mischung wurde dann für die folgende Verzuckerungsreaktion auf 61 °C gebracht. Der pH wurde durch Zugabe von 50%iger Schwefelsäure auf 4,3 eingestellt. Im Verlauf der Reaktion wurde der pH bei diesem Wert gehalten. Die Temperatur wurde bei 61 °C gehalten. 5,74 ml (= 1,5 Gew.-% Enzym/Trockenmasse) Dextrozym GA (Novozymes A/S) wurden zugegeben, um die verflüssigte Stärke in Glucose umzuwandeln. Man ließ die Reaktion eine Stunde fortschreiten. Zur Inaktivierung des Enzyms erhitzte man die Mischung auf 85 °C. Die heiße Mischung füllte man in sterile Behälter und lagerte diese nach Abkühlung bei 4 °C. Es wurde eine Endkonzentration an Glucose von 420 g/l erzielt.

### II.3b) Roggenmehl (inklusive Cellulase/Hemicellulase-Vorbehandlung)

360 g deionisiertes Wasser wurden in einem Reaktionsgefäß vorgelegt. Unter stetem Rühren ließ man 155 g Roggehmehl langsam in das Wasser einrieseln. Die Temperatur wurde konstant bei 50 °C gehalten. Nach Einstellung des pH auf 5,5 mit 50 gew.%iger wässriger NaOH-Lösung gab man 3,21 ml (= 2,5 Gew.-% Enzym/Trockenmasse) Viscozyme L (Novozymes A/S) zu. Nach 30 min startete man die Zugabe von weiterem Mehl, zunächst gab man 55 g Mehl zu. Nach weiteren 30 min gab man erneut 50 g Mehl hinzu; 30 min später noch einmal 40 g Mehl. 30 min nach der letzten Zugabe konnte mit der Verflüssigung begonnen werden.

1,7 ml CaCl₂ Stammlösung (100 g CaCl₂ x 2 H₂O / l) wurden zugegeben. Nach Einstellung des pH auf 6,5 mit 50 gew.-%iger wässriger NaOH-Lösung gab man 5,0 ml (= 2 Gew.-% Enzym/Trockenmasse) Termamyl 120 L Typ L (Novozymes A/S) zu. Die Maische wurde dann schnell auf 85 °C erhitzt. Hierbei wurde der pH fortwährend kontrolliert und gegebenenfalls angepasst.

Nach Erreichen der endgültigen Temperatur begann man mit der Zugabe von weiterem Mehl, zunächst von 60 g Mehl. Zusätzlich gab man der Maische 0,13 ml CaCl₂ Stammlösung zu, um die Ca²⁺-Koncentration bei 70 ppm zu halten. Während der Zugabe hielt man die Temperatur konstant bei 85 °C. Man wartete mindestens 10 min ab, um eine vollständige Reaktion zu gewährleisten, bevor man eine weitere Portion (40 g Mehl und 0,1 ml CaCl₂ Stammlösung) zugab. Man gab 1,1 ml Termamyl zu; anschließend gab man eine weitere Portionen (40 g Mehl und 0,1 ml CaCl₂ Stammlösung) zu. Es wurde ein Gehalt an Trockenmasse von 55 Gew.-% erreicht. Nach der Zugabe erhöhte man die Temperatur auf 100 °C und kochte die Maische 10 min.

Man entnahm eine Probe und kühlte diese auf Raumtemperatur. Nach Verdünnung der Probe mit deionisiertem Wasser (etwa 1:10) gab man einen Tropfen konzentrierte Lugolsche Lösung (Mischung von 5 g Iod und 10 g Kaliumiodid pro Liter) zu. Eine tiefblaue Färbung zeigte einen Gehalt an verbleibender Stärke an; eine Braunfärbung trat ein, wenn die Stärke vollständig hydrolysiert worden war. Wenn der Test einen Anteil verbleibender Stärke anzeigte, wurde die Temperatur erneut auf 85 °C erniedrigt und konstant gehalten. Man gab weitere 1,1 ml Termamyl zu, bis die lod-Stärke-Reaktion negativ ausfiel.

Die negativ auf Stärke getestete Mischung wurde dann für die folgende Verzuckerungsreaktion auf 61 °C gebracht. Der pH wurde durch Zugabe von 50%iger Schwefelsäure auf 4.3 eingestellt. Im Verlauf der Reaktion wurde der pH bei diesem Wert gehalten. Die Temperatur wurde bei 61 °C gehalten. 5,74 ml (= 1.5 Gew.-% Enzym/Trockenmasse) Dextrozyme GA (Novozymes A/S) wurden zugegeben, um die verflüssigte Stärke in Glucose umzuwandeln. Man ließ die Reaktion eine Stunde fortschreiten. Zur Inaktivierung des Enzyms erhitzte man die Mischung auf 85 °C. Die heiße Mischung füllte man in sterile Behälter und lagerte diese nach Abkühlung bei 4 °C. Es wurde eine Endkonzentration an Glucose von 370 g/l erzielt.

### II.3c) Weizenmehl (inklusive Xylanase-Vorbehandlung)

360 g deionisiertes Wasser wurden in einem Reaktionsgefäß vorgelegt. Das Wasser wurde auf 55 °C erhitzt und der pH mit 50 gew.-%iger wässriger NaOH-Lösung auf 6,0 eingestellt. Nach Einstellung von Temperatur und pH gab man 3,21 ml (= 2,5 Gew.-% Enzym/Trockenmasse) Shearzyme 500L (Novozymes A/S) zu. Unter stetem Rühren ließ man 155 g Weizenmehl langsam in die Lösung einrieseln. Die Temperatur und der pH wurden konstant gehalten. Nach 30 min startete man die Zugabe von weiterem Mehl, zunächst gab man 55 g Mehl zu. Nach weiteren 30 min gab man 50 g Mehl hinzu; 30 min später noch einmal 40 g Mehl. 30 min nach der letzten Zugabe konnte mit der Verflüssigung begonnen werden.

Die Verflüssigung und Verzuckerung wurden wie unter II.3b beschrieben durchgeführt. Es wurde eine Endkonzentration an Glucose von 400 g/l erzielt.

### III. Stamm ATCC13032 lysCfbr

In einigen der nachfolgenden Beispiele wurde ein modifizierter Stamm von Corynebacterium glutamicum eingesetzt; der unter der Bezeichnung ATCC13032 lysCfbr in der WO 05/059144 beschrieben wurde.

### Beispiel 1

Je ein Mais-, Weizen-, und Roggenmehlhydrolysat wurde, wie unter 1) unten beschrieben, hergestellt. Der Gesamtzuckergehalt in jedem dieser Medien wurde mittels Zugabe verschiedener Zuckerlösungen (enthaltend Glucose, Rohzucker, Melasse) erhöht. Die Medien wurden in Schüttelkolbenversuchen unter Verwendung von *Corynebacterium glutamicum* (ATCC13032 lysC^{fbr}) und *Bacillus* PA824 (genaue Beschreibung in der WO 02/061108) als Kohlenstoffquelle eingesetzt.

### 1) Herstellung des Mehlhydrolysats

### a) Maismehlhydrolysat

360 g deionisiertes Wasser wurden in einem Reaktionsgefäß vorgelegt. Unter stetem Rühren ließ man 155 g Maismehl langsam in das Wasser einrieseln.

### - Verflüssigung

Nach Einstellung des pH auf 5,8 mit 50 gew.-%iger wässriger NaOH-Lösung gab man 2,6 ml (= 2 Gew.-% Enzym/Trockenmasse) Liquozyme SC (von Novozymes A/S) zu. Die Maische wurde dann schnell auf 100 °C erhitzt und 10 Minuten gekocht. Hierbei wurde der pH fortwährend kontrolliert und gegebenenfalls angepasst.

Man entnahm eine Probe und kühlte diese auf Raumtemperatur. Nach Verdünnung der Probe mit deionisiertem Wasser (etwa 1:10) gab man einen Tropfen konzentrierte Lugolsche Lösung (Mischung von 5 g Iod und 10 g Kaliumiodid pro Liter) zu. Eine tiefblaue Färbung zeigte hierbei einen Gehalt an verbleibender Stärke an; eine Braunfärbung trat ein, wenn die Stärke vollständig hydrolysiert wurde.

### -Verzuckerung

Die negativ auf Stärke getestete Mischung wurde dann für die folgende Verzuckerungsreaktion auf 61 °C gebracht. Der pH wurde durch Zugabe von 50%iger Schwefelsäure auf 4,3 eingestellt. Im Verlauf der Reaktion wurde der pH bei diesem Wert gehalten: Die Temperatur wurde bei 61 °C gehalten. 2,0 ml (= 1,5 Gew.-% Enzym/Trockenmaste) Dextrozym GA (von Novozymes A/S) wurden zugegeben, um die verflüssigte Stärke in Glucose umzuwandeln. Man ließ die Reaktion eine Stunde fortschreiten. Zur Inaktivierung des Enzyms erhitzte man die Mischung auf 85 °C. Die heiße Mischung füllte man in sterile Behälter und lagerte diese nach Abkühlung bei 4 °C.

### b) Weizenmehlhydrolysat

### - Xylanasevorbehandlung

360 g deionisiertes Wasser wurden in einem Reaktionsgefäß vorgelegt. Das Wasser wurde auf 55 °C erhitzt und der pH mit 50 gew.-%iger wässriger NaOH-Lösung auf 6,0 eingestellt. Nach Einstellung von Temperatur und pH gab man 3,21 ml (= 2,5 Gew.-% Enzym/Trockenmasse) Shearyme 500L (von Novozymes A/S) zu. Unter stetem Rühren ließ man 155 g Weizenmehl langsam in die Lösung einrieseln. Die Temperatur und der pH wurden konstant gehalten. Nach 30 min konnte mit der Verflüssigung begonnen werden.

Die Verflüssigung und Verzuckerung wurden wie unter 1a) beschrieben durchgeführt.

### c) Roggenmehlhydrolysat

### Cellulase-/Hemicellulasevorbehandlung

360 g deionisiertes Wasser wurden in einem Reaktionsgefäß vorgelegt. Unter stetem Rühren ließ man 155 g Roggenmehl langsam in das Wasser einrieseln. Die Temperatur wurde konstant bei 50 °C gehalten. Nach Einstellung des pH auf 5,5 mit 50%iger Schwefelsäure gab man 3,21 ml (= 2,5 Gew.-% Enzym/Trockenmasse) Viscozyme L (von Novozymes A/S) zu. Nach 30 min konnte mit der Verflüssigung begonnen werden.

Die Verflüssigung und Verzuckerung wurden wie unter 1a) beschrieben durchgeführt.

### 2) Herstellung des Inokulums

### a) für Corynebacterium glutamicum

Die Zellen wurden nach dem Ausstreichen auf sterilem CM+CaAc-Agar (Zusammensetzung: siehe Tabelle 1; 20 min bei 121 °C) über Nacht bei 30 °C inkubiert. Anschließend wurden die Zellen von den Platten abgekratzt und in Saline resuspendiert. 25 ml des Mediums (siehe Tabelle 4) in 250 ml Erlenmeyerkolben mit zwei Schikanen wurden jeweils mit einer solchen Menge der so hergestellten Zellsuspension angeimpft, dass die optische Dichte einen Wert von OD610 = 0,5 bei 610 nm erreichte.

**Tabelle 1: Zusammensetzung der CM+CaAc-Agarplatten**

| Konzentration | Inhaltsstoff |
|---|---|
| 10,0 g/l | D-Glucose |
| 2,5 g/l | NaCl |
| 2,0 g/l | Harnstoff |
| 5,0 g/l | Bacto Pepton (Difco) |
| 5,0 g/l | Hefeextrakt (Difco) |
| 5,0 g/l | Beef Extract (Difco) |
| 20,0 g/l | Casaminoacids |
| 20,0 g/l | Agar |

### b) für Bacillus

42 ml des Vorkulturmediums (siehe Tabelle 2) in 250 ml Erlenmeyerkolben mit zwei Schikanen wurden jeweils mit 0,4 ml einer Gefrierkultur angeimpft und 24 h bei 43 °C unter Bewegen (250 Upm) in einem befeuchteten Schüttelschrank inkubiert.

**Tabelle 2: Zusammensetzung des Vorkulturmediums**

| Inhaltsstoff | Konzentration |
|---|---|
| Maltose | 28,6 g/l |
| Sojamehl | 19,0 g/l |
| (NH₄)₂SO₄ | 7,6 g/l |
| Mononatriumglutamat | 4,8 g/l |
| Natriumcitrat | 0,95 g/l |
| FeSO₄ x 7 H₂O | 9,5 mg/l |
| MnCl₂ x 4 H₂O | 1,9 mg/l |
| ZnSO₄ x 7 H₂O | 1,4 mg/l |
| CoCl₂ x 6 H₂O | 1,9 mg/l |
| CuSO₄ x 5 H₂O | 0,2 mg/l |
| Na₂MoO₄ x 2 H₂O | 0,7 mg/l |
| K2HPO₄ x 3 H₂O | 15,2 g/l |
| KH₂PO₄ | 3,9 g/l |
| MgCl₂ x 6 H₂O | 0,9 g/l |
| CaCl₂ x 2 H₂O | 0,09 g/l |
| MOPS | 59,8 g/l |
| pH* | 7,2 |

| | |
|---|---|
| * mit verdünnter wässriger KOH-Lösung einzustellen | |

42 ml des Hauptkulturmediums (siehe Tabelle 6) in 250 ml Erlenmeyerkolben mit zwei Schikanen wurden jeweils mit 1 ml Vorkultur angeimpft

### 3) Herstellung der Fermentationsbrühe

### a) für Corynebacterium glutamicum

Die Zusammensetzung des Kolbenmediums ist in Tabelle 4 aufgeführt. Es sollte eine Anfangszuckerkonzentration von 60 g/l vorliegen. Dabei stammte die Hälfte des Zuckers aus dem Hydrolysat (Fermentationsmedium (1)), die andere Hälfte wurde als Zuckerlösung zugesetzt. Hierzu wurde eine Mischung aus Hydrolysat und Zuckerlösung hergestellt und dem Kolbenmedium zugegeben. Im Kontrollmedium wurde eine entsprechende Menge Glucoselösung verwendet.

### - Herstellung der Mehlhydrolysate mit Zuckerzusatz

Es wurden folgende Lösungen angesetzt (siehe Tabelle 3):

**Tabelle 3: Ansatz Mehlhydrolysate mit Zuckerzusatz**

| Mehlhydrolysat | Glucosekonzentration im Hydrolysat [g/l] | Hydrolysat pro Liter Ansatz [ml] | Zuckerlösung | Konzentration in der Zucker lösung [g/l] | Zuckerlösung pro Liter Ansatz [ml] |
|---|---|---|---|---|---|
| Mais 30% | 250,0 | 240 | Glucose | 626 | 96 |
| Mais 30% | 250,0 | 240 | Rohzucker | 639 | 94 |
| Weizen 30% | 258,9 | 232 | Glucose | 626 | 96 |
| Weizen 30% | 258,9 | 232 | Rohzucker | 639 | 94 |
| Roggen 30% | 217,9 | 275 | Glucose | 626 | 96 |
| Roggen 30% | 217,9 | 275 | Rohzucker | | 94 |

**Tabelle 4: Kolbenmedium**

| | |
|---|---|
| Mehlhydrolysat mit Zuckerlösung | 500 ml/l |
| (NH₄)₂SO₄ | 20 g/l |
| Harnstoff | 5 g/l |
| KH₂PO₄ | 0,113 g/l |
| K2HPO₄ | 0,138 g/l |
| ACES | 52 g/l |
| MOPS | 21 g/l |
| Citronensäure x H₂O | 0,49 g/l |
| 3,4-Dihydroxybenzoesäure | 3,08 mg/l |
| NaCl | 2,5 g/l |
| KCl | 1 g/l |
| MgSO₄ x 7 H₂O | 0,3 g/l |
| FeSO₄ x 7H₂O | 25 mg/l |
| MnSO₄ x 4 - 6 H₂O | 5 mg/l |
| ZnCl₂ | 10 mg/l |
| CaCl₂ | 20 mg/l |
| H₃BO₃ | 150 µg/l |
| CoCl₂ x 6 H₂O | 100 µg/l |
| CuCl₂ x 2 H₂O | 100 µg/l |
| NiSO₄ x 6 H₂O | 100 µg/l |
| Na₂MoO₄ x 2 H₂O | 25 µg/l |
| Biotin (Vit. H) | 1050 µg/l |
| Thiamin x HCl (Vit B₁) | 2100 µg/l |
| Nicotinamid | 2,5 mg/l |
| Pantothensäure | 125 mg/l |
| Cyanocobalamin (Vit B₁₂) | 1 µg/l |
| 4-Aminobenzoesäure (PABA; Vit. H₁) | 600 µg/l |
| Folsäure | 1,1 µg/l |
| Pyridoxin (Vit. B₆) | 30 µg/l |
| Riboflavin (Vit. B₂) | 90 µg/l |
| Mehlhydrolysat mit Zuckerlösung | 500 ml/l |
| CSL | 40 ml/l |
| pH* | 6,85 |

| | |
|---|---|
| * mit verdünnter wässriger NaOH-Lösung einzustellen | |

Nach dem Animpfen wurden die Kolben drei Tage bei 30 °C und unter Bewegen (200 Upm) in einem befeuchteten Schüttelschrank inkubiert. Nach dem Abbruch der Fermentation wurde der Gehalt an Lysin per HPLC bestimmt. Die HPLC-Analysen wurden mit Geräten der 1100 Series LC von Agilent durchgeführt. Die Bestimmung der Aminosäurekonzentration erfolgte mittels Hochdruckflüssigkeitschromatographie auf einer Agilent 1100 Series LC System HPLC. Eine Vorsäulenderivatisierung mit ortho-Phthalaldehyd erlaubt die Quantifizierung der gebildeten Aminosäuren, die Auftrennung des Aminosäuregemisches findet auf einer Hypersil AA-Säule (Agilent) statt. Die Ergebnisse sind in Tabelle 5 zusammengestellt.

**Tabelle 5: Mittelwerte**

| Mehlhydrolysat | Zuckerlösung | Lysin [g/l] | Ausbeute* |
|---|---|---|---|
| Mais | Glucose | 12,50 | 0,21 |
| | Rohzucker | 10,64 | 0,19 |
| | Melasse | 10,06 | 0,18 |
| Weizen | Glucose | 10,82 | 0,18 |
| | Rohzucker | 10,14 | 0,18 |
| | Melasse | 9,67 | 0,17 |
| Roggen | Glucose | 10,89 | 0,18 |
| | Rohzucker | 9,59 | 0,16 |
| | Melasse | 9,67 | 0,16 |
| Kontrolle | | 11,54 | 0,20 |

| | | | |
|---|---|---|---|
| * bezogen auf Gesamtglucoseäquivalente | | | |

### b) für Bacillus

Die Zusammensetzung des Kolbenmediums ist in Tabelle 6 aufgeführt. Es sollte eine Anfangsglucosekonzentration von 28,6 g/l vorliegen. Dabei stammte die Hälfte des Zuckers aus dem Hydrolysat, die andere Hälfte wurde als Glucoselösung zugesetzt. Im Kontröllmedium wurde eine entsprechende Menge Glucoselösung verwendet.

**Tabelle 6: Kolbenmedien**

| | | | | |
|---|---|---|---|---|
| Mais | 250 g/l † | 57 ml/l ‡ | | |
| Weizen | 259 g/l † | | 55 ml/l ‡ | |
| Roggen | 218 g/l † | | | 67 ml/l ‡ |
| Glucoselösung (c=626 g/l) | | 23 ml/l | | |
| Sojamehl | | 19,0 g/l | | |
| (NH₄)₂SO₄ | | 7,6 g/l | | |
| Mononatriumglutamat | | 4,8 g/l | | |
| Natriumcitrat | | 0,95 g/l | | |
| FeSO₄ x 7 H₂O | | 9,5 mg/l | | |
| MnCl₂ x 4 H₂O | | 1,9 mg/l | | |
| ZnSO₄ x 7 H₂O | | 1,4 mg/l | | |
| CoCl₂ x 6 H₂O | | 1,9 mg/l | | |
| CuSO₄ x 5 H₂O | | 0,2 mg/l | | |
| Na₂MoO₄ x 2 H₂O | | 0,7 mg/l | | |
| K2HPO₄ x 3 H₂O | | 15,2 g/l | | |
| KH₂PO₄ | | 3,9 g/l | | |
| MgCl₂ x 6 H₂O | | 0,9 g/l | | |
| CaCl₂ x 2 H₂O | | 0,09 g/l | | |
| MOPS | | 59,8 g/l | | |
| pH* | | 7,2 | | |

| | | | | |
|---|---|---|---|---|
| * mit verdünnter wässriger NaOH-Lösung einzustellen t Glucosekonzentration im Hydrolysat ‡ benötigte Einwaage an Hydrolysat pro Liter Medium | | | | |

Nach dem Animpfen wurden die Kolben 24 h bei 43 °C und unter Bewegen (250 Upm) in einem befeuchteten Schüttelschrank inkubiert. Nach dem Abbruch der Fermentation wurde der Gehalt an Glucose und Pantothensäure per HPLC bestimmt. Die Glucose-bestimmung erfolgte mit Hilfe einer Aminex HPX-87H Säule von Bio-Rad. Die Bestimmung der Pantothensäurekonzentration erfolgte über die Auftrennung auf einer Aqua C18-Säule (Phenomenex).

Die Ergebnisse sind in Tabelle 7 zusammengestellt.

**Tabelle 7: Mittelwerte nach 24 h**

| | Pantothensäure, t=24h [g/l] | Ausbeute [g Pantothensäure / g Glucose] |
|---|---|---|
| Mais | 2,7 | 0,09 |
| Weizen | 2,4 | 0,08 |
| Roggen | 2,7 | 0,09 |
| Kontrolle | 2,7 | 0,09 |

## Patentansprüche

1. Verfahren zur Herstellung von Lysin durch Fermentation, umfassend die folgenden Schritte:
a1) Vermahlen von Getreidekörnern als Stärkequelle unter Erhalt eines Mahlguts, das wenigstens 50 Gew.% der in den vermahlenen Getreidekörnern enthaltenen nicht-stärkehaltigen festen Bestandteile enthält, wobei der Anteil der nicht-stärkehaltigen festen Bestandteile im Mahlgut wenigstens 15 Gew.-% beträgt;
a2) Suspendieren des Mahlguts in einer wässrigen Flüssigkeit und Hydrolyse des Stärkeanteils im Mahlgut durch enzymatisches Verflüssigen und gegebenenfalls anschließendes Verzuckern, wobei man eine erste Mono- oder Oligosaccharide enthaltende Flüssigkeit (1) erhält, welche die nicht-stärkehaltigen festen Bestandteile des Mahlguts enthält und worin die Gesamtkonzentration an Mono-, Di- und Oligosacchariden im Bereich von 100 bis 400 g/kg liegt; und
b) Zugabe Mono- oder Oligosaccharide enthaltener Flüssigkeit (1) zusammen mit verstoffwechselbaren Mono-, Di- und/oder Oligosacchariden oder einer Zusammensetzung, die verstoffwechselbare Mono-, Di- und und/oder Oligosaccharide in einer Konzentration von wenigstens 50 Gew.% enthält und die im Wesentlichen frei ist von in Wasser unlöslichen Feststoffen, zu einem Fermentationsmedium, das einen Mikroorganismus enthält, welcher zur Überproduktion des Lysins befähigt ist, unter Fermentationsbedingungen,
wobei man die Gesamtkonzentration an Mono-, Di- und Oligosacchariden in der ersten Flüssigkeit (1) durch Zugabe von verstoffwechselbaren Mono-, Diund/oder Oligosacchariden oder durch Zugabe von einem Medium, das verstoffwechselbare Mono-, Di- und/oder Oligosaccharide in einer Konzentration von wenigstens 50 Gew.% enthält und das im Wesentlichen frei ist von in Wasser unlöslichen Feststoffen, um wenigstens 50 g/kg erhöht.

2. Verfahren nach Anspruch 1, wobei die Gesamtkonzentration an Mono-, Di- und Oligosacchariden der in Schritt a2) erhaltenen Flüssigkeit (1) im Bereich von 150 bis 350 g/kg liegt.

3. Verfahren nach Anspruch 1 oder 2, wobei die durch Zugabe der Flüssigkeit (1) in die Fermentation eingebrachte Menge an Mono-, Di- und/oder Oligosacchariden, 40 bis 95 Gew.% der Gesamtmenge der in die Fermentation eingebrachten Menge an Mono-, Di- und Oligosacchariden ausmacht.

4. Verfahren nach Anspruch 1, wobei man die Gesamtkonzentration an Mono-, Diund Oligosacchariden in der Flüssigkeit (1) auf einen Wert im Bereich von 450 bis 600 g/kg erhöht.

5. Verfahren nach einem der vorhergehenden Ansprüche, wobei man als Zusammensetzung, die verstoffwechselbare Mono- oder Oligosaccharide enthält, ein Glukose und/oder Saccharose und gegebenenfalls Dextrine enthaltendes Nebenprodukt der Zuckerherstellung einsetzt.

6. Verfahren nach Anspruch 5, wobei man als Zusammensetzung, die verstoffwechselbare Mono-, Di- und/oder Oligosaccharide enthält, Melasse aus der Rübenzuckerherstellung einsetzt.

7. Verfahren nach einem der vorherigen Ansprüche, wobei man in Schritt a2) mindestens eine Teilmenge des aus Schritt a1) erhaltenen Mahlguts durch kontinuierliche oder diskontinuierliche Zugabe zur wässrigen Flüssigkeit unter Hydrolysebedingungen hydrolysiert.

8. Verfahren nach einem der Ansprüche 1 bis 6, wobei man in Schritt a2) die Suspension des Mahlguts durch Einbringen von Wasserdampf in die Suspension auf Temperaturen oberhalb der Verkleisterungstemperatur der im Mahlgut enthaltenen Stärke erhitzt.

9. Verfahren nach einem der vorhergehenden Ansprüche, wobei man Lysin aus der Fermentationsbrühe abreichert oder isoliert und anschließend die flüchtigen Bestandteile der Fermentationsbrühe weitgehend entfernt, wobei man eine feste oder halbfeste Proteinzusammensetzung erhält.

10. Verfahren nach einem der Ansprüche 1 bis 8, wobei man die flüchtigen Bestandteile der Fermentationsbrühe ohne vorherige Isolierung oder Abreicherung eines nichtflüchtigen mikrobiellen Stoffwechselprodukts, und gegebenenfalls ohne vorherige Abtrennung fester Bestandteile, mindestens teilweise entfernt, wobei man eine feste Formulierung eines nichtflüchtigen mikrobiellen Stoffwechselprodukts erhält.

## Claims

1. A process for the fermentative production of lysine, comprising the following steps:
a1) milling cereal kernels as starch feedstock, thus obtaining a millbase which comprises at least 50% by weight of the nonstarchy solid constituents present in the milled cereal kernels, the proportion of the nonstarchy solid constituents in the millbase being at least 15% by weight;
a2) suspending the millbase in an aqueous liquid and hydrolysis of the starch portion in the millbase by enzymatic liquefaction and, optionally, subsequent saccharification, whereby a first liquid (1) which comprises mono- or oligosaccharides is obtained, which liquid comprises the nonstarchy solid constituents of the millbase and in which the total mono-, di- and oligosaccharide concentration is in the range of from 100 to 400 g/kg; and
b) addition of liquid (1) which comprises mono- or oligosaccharides together with metabolizable mono, di- and/or oligosaccharides or together with a composition which comprises metabolizable mono-, di- and/or oligosaccharides in a concentration of at least 50% by weight and which is essentially free from solids which are insoluble in water to a fermentation medium comprising a microorganism which is capable of overproducing the lysine under fermentation conditions,
wherein the total mono-, di- and oligosaccharide concentration in the first liquid (1) is increased by at least 50 g/kg by adding metabolizable mono-, di-and/or oligosaccharides or by adding a medium which comprises metabolizable mono-, di- and/or oligosaccharides in a concentration of at least 50% by weight and which is essentially free from solids which are insoluble in water.

2. The process according to claim 1, wherein the total mono-, di- and oligosaccharide concentration of the liquid (1) obtained in step a2) is in the range of from 150 to 350 g/kg.

3. The process according to claim 1 or 2, wherein the amount of mono-, di- and/or oligosaccharides introduced into the fermentation by addition of the liquid (1) accounts for 40 to 95% by weight of the total amount of mono-, di- and oligosaccharides introduced into the fermentation.

4. The process according to claim 1, wherein the total mono-, di- and oligosaccharide concentration in the liquid (1) is increased to a value in the range of from 450 to 600 g/kg.

5. The process according to any of the preceding claims, wherein the employed composition which comprises metabolizable mono- or oligosaccharides is a by-product of sugar production comprising glucose and/or sucrose and, possibly, dextrins.

6. The process according to claim 5, wherein the employed composition comprising metabolizable mono-, di- and/or oligosaccharides is molasses from the beet sugar production.

7. The process according to any of the preceding claims, wherein, in step a2), at least a portion of the mill base obtained in step a1) is hydrolyzed by adding it, continuously or batchwise, to the aqueous liquid under hydrolysis conditions.

8. The process according to any of claims 1 to 6, wherein, in step a2), the suspension of the millbase is heated above the gelatinization temperature of the starch present in the millbase by introducing steam into the suspension.

9. The process according to any of the preceding claims, wherein lysine is depleted or isolated from the fermentation liquor and the volatile constituents of the fermentation liquor are subsequently substantially removed, a solid or semisolid protein composition being obtained.

10. The process according to any of claims 1 to 8, wherein at least some of the volatile constituents of the fermentation liquor are removed without previous isolation or depletion of a nonvolatile microbial metabolite and, if appropriate, without previous removal of solid constituents, a solid formulation of a nonvolatile microbial metabolite being obtained.

## Revendications

1. Procédé pour la production de lysine par fermentation, comprenant les étapes suivantes :
a1) broyage de grains de céréale en tant que source d'amidon, avec obtention d'un produit de broyage qui contient au moins 50 % en poids des composants solides ne contenant pas d'amidon, contenus dans les grains de céréale broyés, la proportion des composants solides ne contenant pas d'amidon dans le produit de broyage étant d'au moins 15 % en poids ;
a2) mise en suspension du produit de broyage dans un liquide aqueux et hydrolyse de la fraction amidon dans le produit de broyage par liquéfaction enzymatique et éventuellement saccharification subséquente, en obtenant un premier liquide (1) contenant des mono- ou oligosaccharides, qui contient les composants solides ne contenant pas d'amidon du produit de broyage et dans lequel la concentration totale de mono-, di- et oligosaccharides se situe dans la plage de 100 à 400 g/kg ; et
b) addition du liquide (1) contenant des mono- ou oligosaccharides, conjointement avec des mono-, di- et/ou oligosaccharides métabolisables ou avec une composition qui contient des mono-, di- et/ou oligosaccharides métabolisables à une concentration d'au moins 50 % en poids et qui est essentiellement exempte de matières solides insolubles dans l'eau, à un milieu de fermentation qui contient un micro-organisme qui est apte à la surproduction de la lysine, dans des conditions de fermentation,
dans lequel on augmente d'au moins 50 g/kg la concentration totale de mono-, di- et oligosaccharides dans le premier liquide (1) par addition de mono-, di-et/ou oligosaccharides métabolisables ou par addition d'un milieu qui contient des mono-, di- et/ou oligosaccharides métabolisables à une concentration d'au moins 50 % en poids et qui est essentiellement exempt de matières solides insolubles dans l'eau.

2. Procédé selon la revendication 1, dans lequel la teneur totale en mono-, di- et oligosaccharides du liquide (1) obtenu dans l'étape a2) se situe dans la plage de 150 à 350 g/kg.

3. Procédé selon la revendication 1 ou 2, dans lequel la quantité de mono-, di- et/ou oligosaccharides introduite par addition du liquide (1) dans la fermentation représente 40 à 95 % en poids de la quantité totale de la quantité de mono-, di- et oligosaccharides introduite dans la fermentation.

4. Procédé selon la revendication 1, dans lequel on augmente la concentration totale de mono-, di- et oligosaccharides dans le liquide (1) jusqu'à une valeur dans la plage de 450 à 600 g/kg.

5. Procédé selon l'une quelconque des revendications précédentes, dans lequel en tant que composition qui contient des mono- ou oligosaccharides métabolisables on utilise un sous-produit de la fabrication du sucre, contenant du glucose et/ou du saccharose et éventuellement des dextrines.

6. Procédé selon la revendication 5, dans lequel en tant que composition qui contient des mono-, di-et/ou oligosaccharides métabolisables on utilise de la mélasse provenant de la fabrication du sucre de betterave.

7. Procédé selon l'une quelconque des revendications précédentes, dans lequel on hydrolyse dans l'étape a2) au moins une quantité partielle du produit de broyage obtenu dans l'étape a1), par addition continue ou discontinue au liquide aqueux dans des conditions d'hydrolyse.

8. Procédé selon l'une quelconque des revendications 1 à 6, dans lequel on chauffe dans l'étape a2) la suspension du produit de broyage, par introduction de vapeur d'eau dans la suspension, jusqu'à des températures supérieures à la température de gélatinisation de l'amidon contenu dans le produit de broyage.

9. Procédé selon l'une quelconque des revendications précédentes, dans lequel on isole ou diminue la concentration de la lysine du bouillon de fermentation et ensuite on élimine dans une large mesure les composants volatils du bouillon de fermentation, de sorte qu'on obtient une composition de protéine solide ou semi-solide.

10. Procédé selon l'une quelconque des revendications 1 à 8, dans lequel on élimine au moins en partie les composants volatils du bouillon de fermentation sans isolement préliminaire ni diminution préliminaire de la concentration d'un produit métabolique microbien non volatil, et éventuellement sans séparation préliminaire de composants solides, de sorte qu'on obtient une composition solide d'un produit métabolique microbien non volatil.
